# EUROPEAN PATENT SPECIFICATION

(11) **EP 4 395 747 B1**
(45) Date of publication and mention of the grant of the patent: **04.11.2026**
(21) Application number: 22863798.9
(22) Date of filing: 30.08.2022
(51) Int. Cl.: A61K 9/127, A61P 19/02, A61L 12/00, C11D 3/00

(54) **STERILE LIPOSOME-CONTAINING COMPOSITIONS AND ARTICLES**
STERILE LIPOSOMHALTIGE ZUSAMMENSETZUNGEN UND ARTIKEL
COMPOSITIONS ET ARTICLES CONTENANT DES LIPOSOMES STÉRILES

(30) Priority: 31.08.2021 US 202163238831 P; 11.11.2021 US 202163278123 P; 20.06.2022 US 202263353657 P
(43) Date of publication of application: 10.07.2024
(73) Proprietor: Liposphere Ltd., 5400804 Givat Shmuel (IL)
(72) Inventor: GOLDBERG, Ronit, 5442802 Givat Shmuel (IL); JAHN, Sabrina, High Pitfold Hindhead GU26 6BN (GB)
(74) Representative: Regimbeau
(86) International application number: PCT/IL2022/050951
(87) International publication number: WO 2023/031928

(56) References cited:
- WO-A1-2015/193887
- WO-A1-2015/193888
- WO-A1-2016/051413
- WO-A1-2017/109784
- WO-A1-2018/150429
- WO-A1-2018/150429
- WO-A1-99/33940

## Description

### FIELD AND BACKGROUND OF THE INVENTION

The present invention relates to sterile liposomal compositions and process for their preparation. Advantageously the compositions are usable, *inter alia,* for treating joint disorders, wherein the treatment comprises intra-articular administration of the composition, such as osteoarthritis.

Osteoarthritis, the most common form of arthritis, is a degenerative joint disease that results from breakdown of articular cartilage and underlying bone, with the most common symptoms being joint pain and stiffness. Conventional treatments include analgesics and anti-inflammatory drugs, and surgical operations such as bone fusion (e.g., in ankle arthritis) and joint replacement.

In early osteoarthritis, no clear cartilage lesions or combined abnormalities are observed which need to be addressed surgically. Several treatments have been proposed and are currently used in clinical practice for patients without clear osteoarthritis signs but ascribable to the phase defined as early osteoarthritis [Luyten et al., Knee Surg Sports Traumatol Arthrosc 2012, 20:401-406]. Available non-surgical treatments for management of early osteoarthritis include the use of non-pharmacological modalities, such as lifestyle adaptations, exercise, and physical therapy, dietary supplements and pharmacological therapies, such as pain, anti-inflammatory, or slow-acting drugs, as well as the local delivery of various substances through a minimally invasive injective approach [Kon et al., Knee Surg Sports Traumatol Arthrosc 2012, 20:436-449].

Viscosupplementation involves intra-articular injections of hyaluronic acid (HA), a glycosaminoglycan that provides joint lubrication and shock absorbance, and acts as the backbone for the proteoglycans of the extracellular matrix. In normal adult knees, HA concentration ranges from 2.5 to 4.0 mg/ml, whereas in osteoarthritis, the HA concentration decreases by 33-50 % [Kon et al., Knee Surg Sports Traumatol Arthrosc 2012, 20:436-449]. However, there is no evidence which clearly shows that viscosupplementation influences the natural progression of OA; whereas some reports show serum and urine biomarker changes, others show no changes in cartilage structural composition [Conrozier et al., J Orthop Res 2012, 30:679-685].

International Patent Application publication WO 2015/193888 describes a method of reducing a friction coefficient of a surface by attaching a water-soluble polymer to the surface, and contacting the water-soluble polymer with liposomes, thereby coating the surface with an amphiphilic lipid; as well as a method for treating a synovial joint disorder associated with increased articular friction.

In general, pharmaceutical compositions intended for injection must be sterilized to which are to be used internally. Steam sterilization (e.g., at 121-134 °C under pressure) is commonly used, as it is nontoxic, dependable and inexpensive, but it is not suitable for heat-sensitive and/or moisture-sensitive materials. Ethylene oxide gas is used to sterilize heat-sensitive and moisture-sensitive materials. Microfiltration with a pore size of 0.22 µm can be used to remove microorganisms, although removal of viruses requires a smaller pore size (e.g., 20-50 nm). Gamma (γ) radiation is highly penetrating and useful for sterilization, but creates a safety hazard. Ultraviolet radiation is safer but less penetrating, suitable for sterilizing surfaces and some transparent objects, and may damage some plastics.

Toh & Chiu [Asian J Pharm Sci 2013, 8:88-95] review the sterilization of liposome compositions, and disclose that filtration may be useful for sterilization, but is costly, inconvenient, and only applicable to liposomal systems below 200 nm in diameter; whereas UV-irradiation, γ-irradiation and steam sterilization may result in degradation of liposomes.

Steam sterilization is also used to sterilize packaged contact lens upon manufacture and in order to reuse contact lenses.

International Patent Application publication WO 2015/193887 describes formulations for use in rinsing, and/or immersing therein, a contact lens, and/or in the treatment of ocular discomfort (e.g., ocular discomfort associated with a contact lens), the formulations comprising at least one water-soluble polymer, liposomes, and an aqueous carrier.

International Patent Application publication WO 2017/109784 describes polymeric compounds comprising a lipid moiety and an ionic polymeric moiety, such as a pMPC (poly(O-(2-methacryloyloxyethyl)phosphorylcholine)) moiety, as well as bilayers and liposomes comprising such a polymeric compound in combination with a bilayer-forming lipid. The bilayers and/or liposomes comprising such polymeric compounds are described as being useful for reducing a friction coefficient of a surface and/or for inhibiting biofilm formation.

Additional background art includes International Patent Application publications WO 2016/051413 and WO 2018/150429.

### SUMMARY OF THE INVENTION

According to an aspect of the invention, there is provided a sterile composition comprising an aqueous carrier and liposomes, and wherein the liposomes comprise:
a) at least one bilayer-forming lipid; and
b) a polymeric compound having the general formula I: 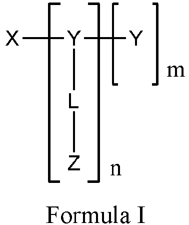
wherein:
m is zero or a positive integer;
n is an integer which is at least 1, wherein when X does not comprise a phosphate group, n is at least 2;
X is a lipid moiety;
Y is a backbone unit which forms a polymeric backbone;
L is absent or is a linking moiety; and
Z has the general formula II: 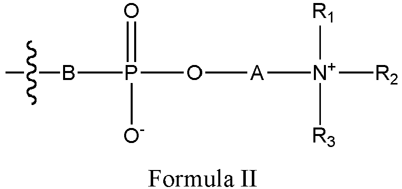
wherein:
   A is a substituted or unsubstituted hydrocarbon;
   B is an oxygen atom or is absent; and
   R₁-R₃ are each independently selected from the group consisting of hydrogen, alkyl, cycloalkyl, heteroalicyclic, aryl and heteroaryl,
      wherein a polydispersity index of said liposomes is no more than 0.2 and/or a zeta potential of said liposomes is in a range of from -40 mV to 40 mV or of from -10 mV to 10 mV,
   and wherein a mean diameter and/or a zeta potential of the liposomes changes by no more than 20 % over the course of 300 days,
   the sterile composition being characterized by:
      an absence of observable microorganism growth upon testing in fluid thioglycollate medium at a temperature in a range of from 30 to 35 °C for up to 3 days, and/or testing the composition in soybean-casein digest medium at a temperature in a range of from 20 to 25 °C for up to 5 days, and/or when tested for sterility according to U.S. Pharmacopeia General Chapter 71; and/or
      a concentration of bacterial endotoxins below a 35 endotoxin units (EU) per ml, as determined using horseshoe crab *(Limulus)* amebocyte lysate according to Chapter 85 of the U.S. Pharmacopeia.

According to an aspect of the invention, there is provided a process for preparing the sterile composition according to the present invention, the process comprising:
(i) providing an aqueous composition comprising the aqueous carrier and liposomes comprising at least one bilayer-forming lipid and the polymeric compound, according to any of the respective embodiments described herein; and
(ii) subjecting the aqueous composition to a temperature of over 100 °C, to thereby obtain the sterile composition.

According to an aspect of the invention, there is provided a process for preparing a sterile article of manufacture having lipids attached to at least a portion of a surface thereof, the process comprising:
(i) contacting at least a portion of a surface of the article of manufacture with an aqueous composition comprising an aqueous carrier and liposomes, to thereby obtain an article of manufacture having lipids attached to at least a portion of a surface thereof, the liposomes comprising:
   a) at least one bilayer-forming lipid; and
   b) a polymeric compound having the general formula I: 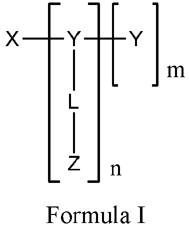 wherein:
      m is zero or a positive integer;
      n is an integer which is at least 1, wherein when X does not comprise a phosphate group, n is at least 2;
      X is a lipid moiety;
      Y is a backbone unit which forms a polymeric backbone;
      L is absent or is a linking moiety; and
      Z has the general formula II:

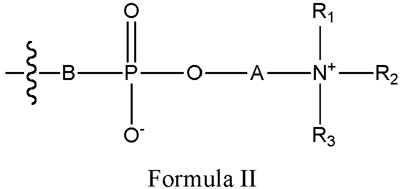

      wherein:
         A is a substituted or unsubstituted hydrocarbon;
         B is an oxygen atom or is absent; and
         R₁-R₃ are each independently selected from the group consisting of hydrogen, alkyl, cycloalkyl, heteroalicyclic, aryl and heteroaryl; and
(ii) subjecting the article of manufacture having lipids attached to at least a portion of surface thereof to a temperature of over 100 °C, to thereby obtain the sterile article of manufacture having lipids attached to at least a portion of a surface thereof,
   the sterile article of manufacture being characterized by:
   an absence of observable microorganism growth upon testing in fluid thioglycollate medium at a temperature in a range of from 30 to 35 °C for up to 3 days, and/or testing the composition in soybean-casein digest medium at a temperature in a range of from 20 to 25 °C for up to 5 days, and/or when tested for sterility according to U.S. Pharmacopeia General Chapter 71; and/or
   a concentration of bacterial endotoxins below a 35 endotoxin units (EU) per ml, as determined using horseshoe crab *(Limulus)* amebocyte lysate according to Chapter 85 of the U.S. Pharmacopeia.

According to an embodiment of the invention, a molar ratio of the bilayer-forming lipid and the polymeric compound is in a range of from 5:1 to 5,000:1.

According to an embodiment of the invention, Y is a substituted or unsubstituted alkylene unit.

According to an embodiment of the invention, Y is a substituted or unsubstituted ethylene unit.

According to an embodiment of the invention, Y has the formula -CR₄R₅-CR₆D-, wherein:
when Y is a backbone unit which is not attached to L or Z, D is R₇; and when Y is a backbone unit which is attached to L or Z, D is a covalent bond or a linking group attaching Y to L or Z, the linking group being selected from the group consisting of -O-, -S-, alkylene, arylene, sulfinyl, sulfonyl, phosphate, phosphonyl, phosphinyl, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino; and
R₄-R₇ are each independently selected from the group consisting of hydrogen, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, azo, phosphate, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino.

According to an embodiment of the invention, B is an oxygen atom.

According to an embodiment of the invention, A is a substituted or unsubstituted hydrocarbon which is from 1 to 4 carbon atoms in length.

According to an embodiment of the invention, R₁-R₃ are each independently hydrogen or C₁₋₄-alkyl.

According to an embodiment of the invention, n is at least 3.

According an embodiment of the invention, n is in a range of from 5 to 150, or from 5 to 100, or from 5 to 50, and m is in a range of from 0 to 50.

According to an embodiment of the invention, the lipid moiety is selected from the group consisting of a fatty acid moiety, a monoglyceride moiety, a diglyceride moiety, a triglyceride moiety, a glycerophospholipid moiety, a sphingolipid moiety, and a sterol moiety.

According to an embodiment of the invention, the lipid moiety comprises at least one fatty acid moiety selected from the group consisting of lauroyl, myristoyl, palmitoyl, stearoyl, palmitoleoyl, oleoyl, and linoleoyl.

According to an embodiment of the invention, X has the general formula III: 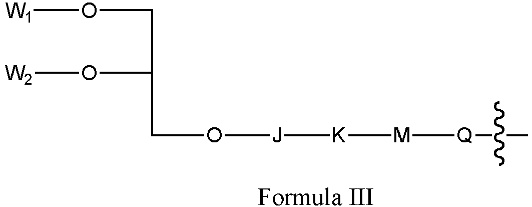 wherein:
W₁ and W₂ are each independently selected from the group consisting of hydrogen, alkyl, alkenyl, alkynyl and acyl, wherein at least one of W₁ and W₂ is not hydrogen;
J is -P(=O)(OH)-O- or absent;
K is a substituted or unsubstituted hydrocarbon from 1 to 10 carbon atoms in length, or absent;
M is a linking group selected from the group consisting of -O-, -S-, amino, sulfinyl, sulfonyl, phosphate, phosphonyl, phosphinyl, carbonyl, thiocarbonyl, urea, thiourea, carbamyl, thiocarbamyl, amido, carboxy, and sulfonamide, or absent; and
Q is a substituted or unsubstituted hydrocarbon from 1 to 10 carbon atoms in length,
wherein when M is absent, K is also absent.

According to an aspect of the invention relating to Formula **III,** J is -P(=O)(OH)-O- and K is selected from the group consisting of an ethanolamine moiety, a serine moiety, a glycerol moiety and an inositol moiety.

According to an embodiment of the invention relating to Formula III, M is amido.

According to an embodiment of the invention relating to Formula III, J and K are absent and M is carbonyl.

According to an embodiment of the invention relating to Formula III, Q is dimethylmethylene (-C(CH₃)₂-).

According to an embodiment of the invention relating to Formula III, at least one of W₁ and W₂ is alkyl, alkenyl, alkynyl or acyl, being from 10 to 30 carbon atoms in length.

According to an embodiment of the invention relating to a sterile composition, the sterile composition is for rinsing, cleaning and/or immersing therein a contact lens.

According to an embodiment of the invention relating to a sterile composition, the aqueous carrier is an ophthalmically acceptable carrier.

According to an embodiment of the invention relating to a sterile composition, the sterile composition comprises an article of manufacture immersed therein.

According to an embodiment of the invention relating to an article of manufacture, the article of manufacture comprises a contact lens.

According to an embodiment of the invention relating to a sterile composition, the sterile composition is capable of improving knee histopathology in an animal model of osteoarthritis following intra-articular injection of the composition.

According to an embodiment, the model is a rat medial meniscal destabilization model.

According to an embodiment of the invention relating to a sterile composition, the sterile composition is for use in the treatment of a synovial joint disorder, wherein the treatment comprises intra-articular administration of the composition.

According to an embodiment of the invention relating to treatment of a synovial joint disorder, the synovial joint disorder is osteoarthritis.

According to an embodiment of the invention relating to treatment of a synovial joint disorder, the treatment is characterized by an improvement in articular physiology, as determined by at least one of Kellgren-Lawrence scale of radiological severity, range of motion of joint, physical activity, and quality of life.

According to an embodiment of the invention relating to treatment of a synovial joint disorder, the treatment is characterized by a reduction in pain.

According an embodiment of the invention relating to a process for preparing a sterile composition, the temperature is in a range of from 121 °C to 134 °C.

According to an embodiment of the invention relating to a process for preparing a sterile composition, the aqueous composition comprising the aqueous carrier and liposomes further comprises an article of manufacture immersed therein, such that upon subjecting the aqueous composition to a temperature of over 100 °C, the article of manufacture immersed in the aqueous composition becomes sterile.

### BRIEF DESCRIPTION OF THE SEVERAL VIEWS OF THE DRAWING(S)

In the drawings:

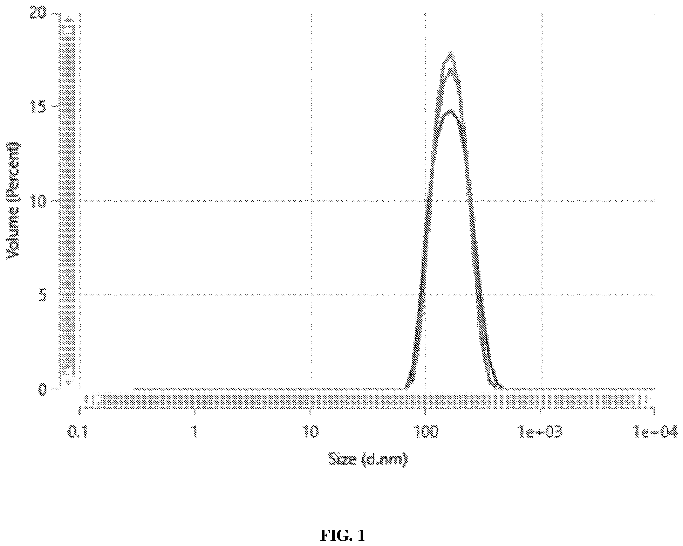

FIG. 1 presents a graph showing the size distribution of liposomes in an exemplary composition (each of the three lines represents a separate measurement).

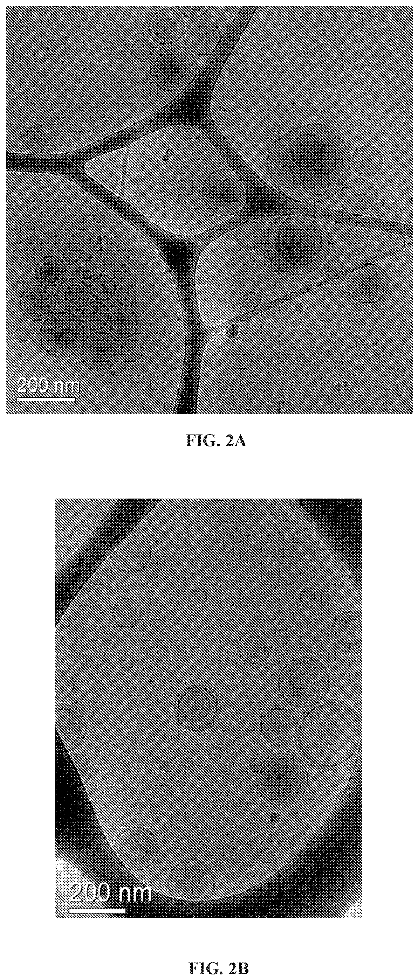

FIG. 2A and 2B present cryo-tunneling electron microscopy images of liposomes in an exemplary composition.

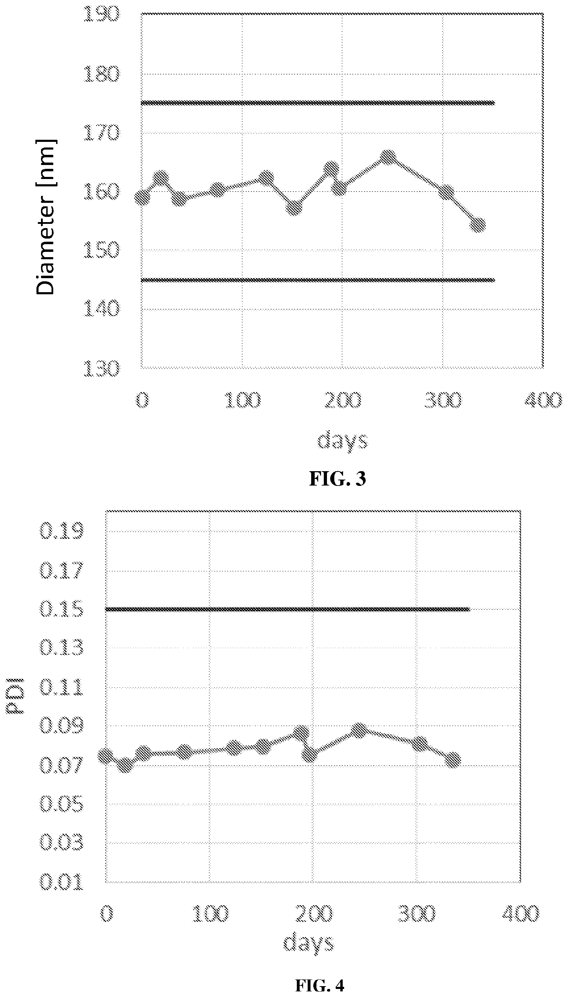

FIG. 3 presents a graph showing the zeta potential of liposomes in an exemplary composition as a function of time (black lines indicate initial value ± 10 %).
FIG. 4 presents a graph showing the polydispersity index (PDI) of liposomes in an exemplary composition as a function of time (black lines indicate PDI = 15 %).

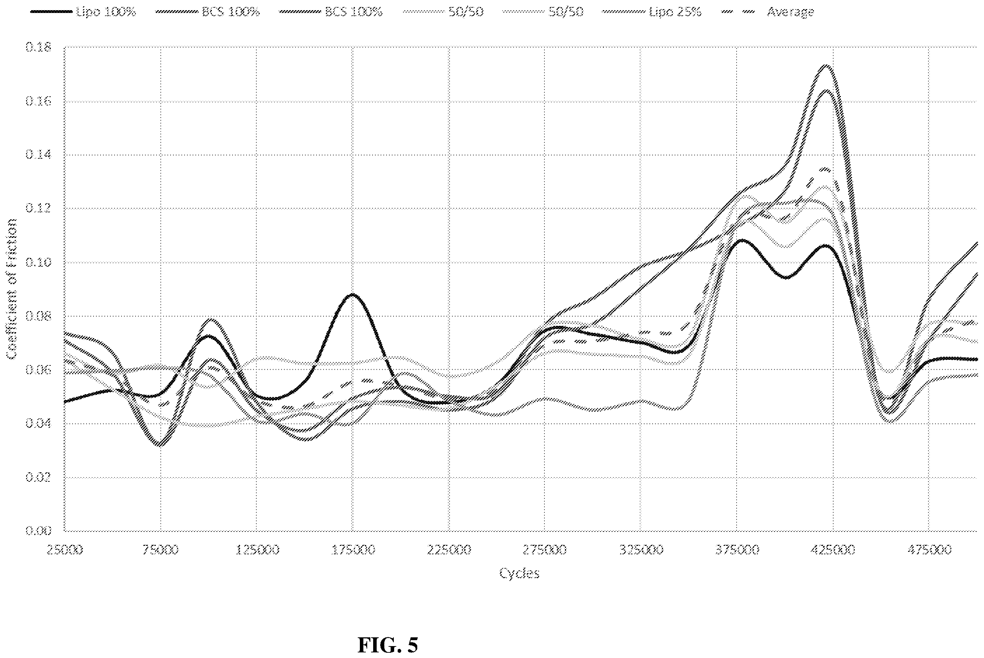

FIG. 5 presents a graph showing the friction coefficient in a simulated knee with a pin-on-disk design over the course of 500,000 cycles, in the presence of an exemplary liposome-containing composition (Lipo 100%), bovine calf serum (BCS 100%) or mixture thereof containing 50 % liposome-containing composition (50/50) or 25 % liposome-containing composition (Lipo 25%); BCS 100% and 50/50 were tested in duplicate; average value for all samples is also shown.

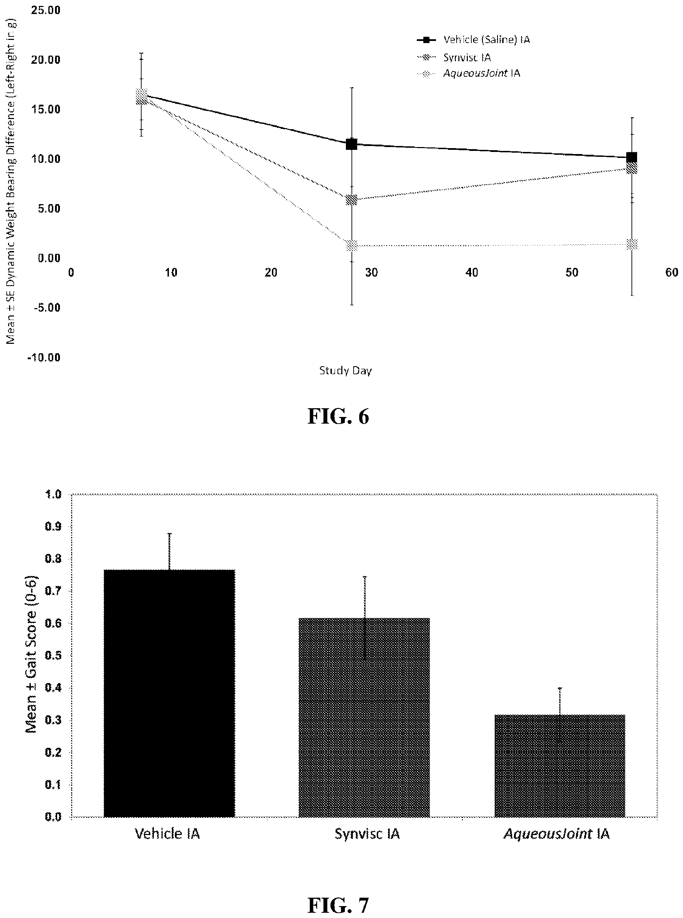

FIG. 6 presents a graph showing dynamic weight bearing difference (Left-Right) in a rat medial meniscal destabilization model of osteoarthritis at the indicated time point post-surgery. Rat were treated at days 7, 21, 35 and 49 post-surgery with AqueousJoint, Synvisc^{®} (a commercial HA injectable product) or saline (vehicle control) via intra-articular (IA) injection.
FIG. 7 presents a graph showing gait score in a rat medial meniscal destabilization model of osteoarthritis on day 10 post-surgery. Rats were treated at day 7 post-surgery with AqueousJoint, Synvisc^{®} (a commercial HA injectable product) or saline (vehicle control) via intra-articular (IA) injection.

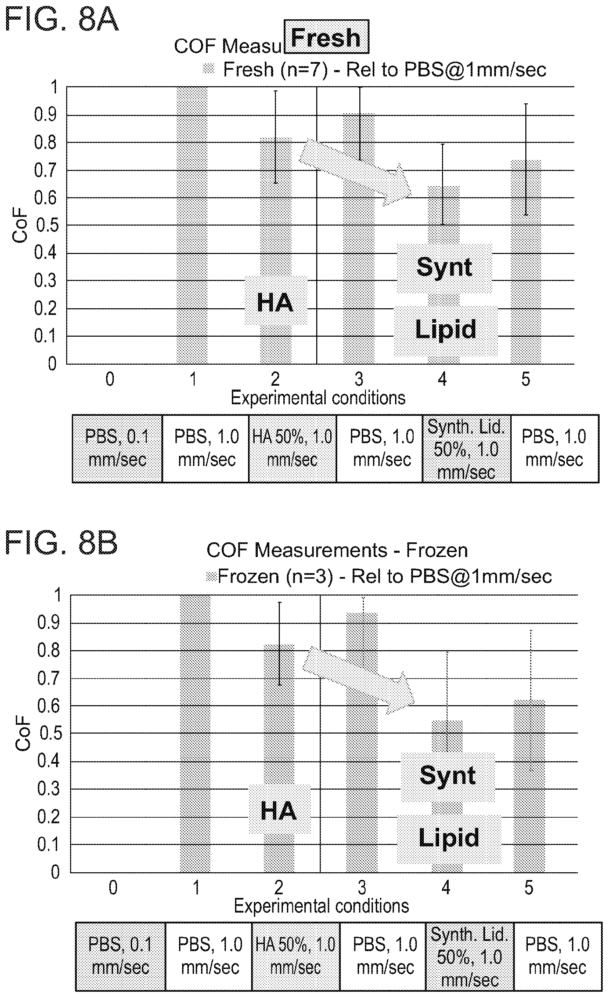

FIGs. 8A-B present a graph showing the changes in the frictional response of fresh (Figure 8A) and frozen (Figure 8B) cartilage exposed to two test solutions - synthetic lipids and hyaluronic acid, respectively. The same sample has been exposed alternatively to either control (buffer) or test solutions (control - test solution - control - test solution - control). The results indicate that the addition of synthetic lipids results in a larger reduction of frictional force than observed with hyaluronic acid.

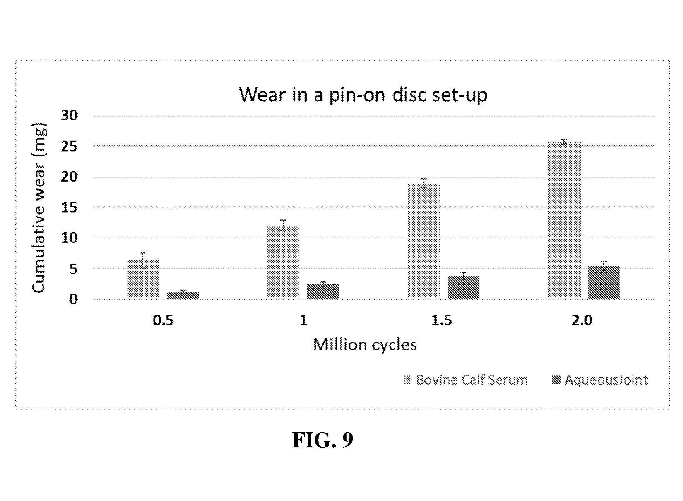

FIG. 9 presents a graph showing a wear test performed using a pin-on-disk setup to demonstrate the wear reduction capabilities of an AqueousJoint formulation as described herein compared to the bovine calf serum standard solution as a control. The results demonstrate that following two million cycles, the AqueousJoint formulation reduced the wear X5 compared to the control solution.

### DESCRIPTION OF SPECIFIC EMBODIMENTS OF THE INVENTION

The present invention, relates to sterile liposomal compositions, their process of preparation and use for treating joint disorders such as osteoarthritis.

While investigating liposome-containing solutions, the present inventors have serendipitously found that compositions comprising liposomes with lipid-derived polymeric compounds formed of a backbone chain composed of backbone units that include both phosphate and ammonium ionic groups, are surprisingly stable towards stabilization by heat treatment, which typically promotes liposome instability, for example, characterized by merging and/or aggregation of liposomes and/or significant increases in liposome size.

As demonstrated in the Examples section that follows, the present inventors have uncovered that compositions including liposomes comprising such a polymeric compound exhibit very little changes in liposome properties upon sterilization, as well as over the course of almost a year thereafter.

Embodiments of the present invention therefore relate to sterile compositions comprising liposomes and preparation of such sterile compositions, as well the use of such sterile compositions for various applications, especially applications wherein sterility is an important feature, such as in treatments of synovial joint disorders (e.g., osteoarthritis) involving articular administration of the composition, and in contact lens solutions and treatment of contact lens surfaces.

### Sterile composition:

According to an aspect of the invention, there is provided a sterile composition comprising an aqueous carrier and liposomes, wherein the liposomes comprise at least one bilayer-forming lipid and a polymeric compound as defined in the present invention.

Herein, the term "sterile" refers to an absence of observable microorganism growth upon subjecting composition to conditions (e.g., medium, incubation temperature) for a suitable period of time, e.g., according to any standard protocol for testing sterility. Optionally, sterility is tested in fluid thioglycollate medium, at a temperature in a range of from 30 to 35 °C for up to 3 days, and/or soybean-casein digest medium (a.k.a. trypticase soy broth or trypticase soy agar), at a temperature in a range of from 20 to 25 °C for up to 5 days; for example, whereby a composition is sterile if no microbial growth is observable in either medium. The fluid thioglycollate medium and/or soybean-casein digest medium content and/or procedures for testing sterility may optionally be as described in U.S. Pharmacopeia General Chapter 71.

According to the present invention, a sterile composition is further characterized by a concentration of bacterial endotoxins below 35 endotoxin units (EU) per ml (e.g., wherein endotoxin units are defined according to the U.S. Pharmacopeia reference standard). Bacterial endotoxin level may be determined using horseshoe crab *(Limulus)* amebocyte lysate (e.g., according to Chapter 85 of the U.S. Pharmacopeia), for example, by comparing with commercially available reference samples containing endotoxins.

According to the present invention, a polydispersity index (PDI) of the liposomes in the sterile composition is no more than 0.2, and optionally no more than 0.15, or no more than 0.1, or even no more than 0.05.

According to the present invention, a mean diameter of the liposomes is in a range of from 20 nm to 1000 nm, optionally from 50 nm to 300 nm.

According to the present invention, a mean diameter of the liposomes is at least 100 nm, for example from 100 to 1000 nm, or from 100 nm to 300 nm. In some embodiments, an average diameter of the liposomes is at least 125 nm, for example from 125 to 1000 nm, or from 125 nm to 300 nm. In some embodiments, an average diameter of the liposomes is at least 150 nm, for example from 150 to 1000 nm, or from 150 nm to 300 nm.

Without being by bound by any particular theory, it is believed that liposome diameters of at least about 100 nm are associated with greater retention times *in vivo,* and thus are particularly suitable for sterile compositions intended for use *in vivo* (according to any of the respective embodiments described herein).

According to the present invention, a mean diameter of liposomes in the sterile composition changes by no more than 20 % over the course of 300 days (e.g., at room temperature), and optionally by no more than 15 % or no more than 10 % or even no more than 5 % over the course of 300 days (e.g., at room temperature).

The mean diameter according to any of the respective embodiments described herein may optionally be an arithmetic mean (a ratio of a sum of values to the number of values) or a Z-average as this term is defined in the art of dynamic light scattering (in brief, an intensity-weighted harmonic mean). In exemplary embodiments, the mean diameter is a Z-average diameter determined by dynamic light scattering.

The polydispersity index (PDI) and/or mean diameter of liposomes in a composition may optionally be determined by dynamic light scattering using a two-parameter fit to the data (e.g., according to ISO 13321 and ISO 22412 standards) for determining PDI and Z-average diameter (e.g., using a commercially available instrument).

According to the present invention, a zeta potential of the liposomes in the sterile composition is in a range of from 40 mV to -40 mV, or from 30 mV to -30 mV, or from 20 mV to -20 mV, or from 15 mV to -15 mV, or from 10 mV to -10 mV (e.g., from 5 mV to -5 mV), optionally in a range of from 0 to -10 mV (e.g., from 0 to -5 mV, or from -3 mV to -5 mV).

Without being bound by any particular theory, it is believed that a zeta potential in a range as indicated, e.g., of from 10 mV to -10 mV, is typically indicative of liposomes which are especially susceptible to instability (e.g., for liposomes which lack a polymeric compound described herein), such that stability of liposomes with such a zeta potential according to embodiments of the invention (e.g., upon sterilization) is particularly remarkable and difficult to achieve for other liposomes with such a zeta potential.

According to the present invention, a zeta potential of liposomes in the sterile composition changes by no more than 20 % over the course of 300 days (e.g., at room temperature or optionally lower), and optionally by no more than 15 % or no more than 10 % or even no more than 5 % over the course of 300 days (e.g., at room temperature or optionally lower). In some such embodiments, a mean diameter of liposomes in the sterile composition changes by no more than 20 % over the course of 300 days (e.g., at room temperature or optionally lower) (according to any of the respective embodiments described herein). In some embodiments, the indicated change in zeta potential over the course of 300 days refers to room temperature. In some embodiments, the indicated change in zeta potential over the course of 300 days refers to 4 °C.

Zeta potential may optionally be determined using any suitable technique known in the art (e.g., using a commercially available instrument), for example, electrophoretic light scattering. The zeta potential of liposomes may be determined by diluting the liposomes in an aqueous salt (e.g., NaCl) solution with a predetermined salt concentration (e.g., 10 µM).

As described herein, the liposome according to embodiments comprises, *inter alia,* at least one bilayer-forming lipid.

Herein, the term "bilayer-forming lipid" encompasses any compound in which a bilayer may form from a pure aqueous solution of the compound, the bilayer comprising two parallel layers of molecules of the compound (referred to as a "lipid").

Typically, the bilayer in a liposome according to the present invention comprises relatively polar moieties of the lipid at the two surfaces of the bilayer, which may optionally comprise an interface with the aqueous solution and/or an interface with a solid surface; and relatively hydrophobic moieties of the lipid at the interior of the bilayer, at an interface between the two layers of lipid molecules which form the bilayer.

Examples of bilayer-forming lipids include glycerophospholipids. Suitable examples of glycerophospholipids include, without limitation, a phosphatidyl ethanolamine, a phosphatidyl serine, a phosphatidyl glycerol and a phosphatidyl inositol.

It is to be appreciated that the polymeric compound comprised by a liposome (according to any of the respective embodiments described herein) may optionally be a bilayer-forming lipid which can form a bilayer *per se* or in combination with one or more additional bilayer-forming lipids.

In some embodiments of any one of the embodiments described herein, the bilayer-forming lipid comprises at least one charged group (e.g., one or more negatively charged groups and/or one or more positively charged groups).

In some embodiments, the bilayer-forming lipid is zwitterionic; comprising both (e.g., an equal number of) negatively charged and positively charged groups (e.g., one of each).

In some embodiments of any of the embodiments described herein, a molar ratio of the bilayer-forming lipid (comprised in addition to the polymeric compound) and the polymeric compound (according to any of the respective embodiments described herein) in the liposome is in a range of from 5:1 to 5,000:1 (bilayer-forming lipid: polymeric compound), optionally in a range of from 10:1 to 2,500:1, optionally in a range of from 25:1 to 1,000:1, and optionally in a range of from 50:1 to 500:1.

A liposome may optionally comprise a single bilayer (e.g., a unilamellar vesicle) or a plurality of bilayers (e.g., a multilamellar vesicle) - wherein each bilayer optionally independently forms a closed vesicle - comprising, for example, concentric bilayer vesicles and/or a plurality of separate bilayer vesicles encompassed by the same bilayer vesicle.

A liposome according to any of the respective embodiments described herein may be approximately spherical in shape or may have any alternative shape, such as an elongated tube and/or a flattened (e.g., sheet-like) shape.

In an embodiment of the present invention, the liposomes, and optionally the sterile composition as a whole, is devoid of a therapeutically active agent.

According to an embodiment of the present invention, the sterile composition comprises a therapeutically active agent, which is optionally incorporated in a liposome and/or on a surface of the liposome. In some such embodiments, the therapeutically active agent is a therapeutically active agent described in International Patent Application publication WO 2018/150429.

Herein, the phrase "therapeutically active agent" refers to any agent (e.g., compounds) having a therapeutic effect, provided that the compound is not a bilayer-forming lipid or polymeric compound comprised by the liposome (according to any of the respective embodiments described herein), as well as to any portion of an agent (e.g., a moiety of a compound) which generates an agent having a therapeutic effect upon release (e.g., upon cleavage of one or more covalent bonds), including a portion of a bilayer-forming lipid or polymeric compound. Thus, the bilayer-forming lipid and polymeric compound *per se* are excluded from the definition of a therapeutically active agent, but a bilayer-forming lipid and/or polymeric compound may optionally comprise generate a therapeutically active agent upon release, in which case the portion of the bilayer-forming lipid and/or polymeric compound which generates the therapeutically active agent is also considered to be a therapeutically active agent as defined herein.

When present, a therapeutically active agent in the sterile composition may be associated with a liposome and/or not associated with a liposome (e.g., dissolved in the carrier). When associated with a liposome, a therapeutically active agent may optionally be attached by a covalent or non-covalent (e.g., electrostatic and/or hydrophobic) bond to a liposome (e.g., to an exterior surface and/or interior surface of a liposome membrane), incorporated within a liposome membrane (e.g., a lipophilic agent which stably partitions to a lipid phase of the liposome), and/or enveloped within a core of a liposome (e.g., a hydrophilic agent in an aqueous compartment of the liposome). The therapeutically active agent may optionally be a moiety covalently attached to a liposome (e.g., attached to a lipid so as to form a lipid-derivative comprising the moiety). Such attachment may be obtained in some embodiments by using techniques known in the art (e.g., amide bond formation).

Preferably, the sterile composition comprises an article of manufacture immersed therein (which, being part of the sterile composition, is also sterile), such as a solid or semi-solid article of manufacture which is commonly packaged together with a liposome-containing aqueous composition. In some embodiments, the article of manufacture is a contact lens, for example, wherein the aqueous carrier and liposomes represent a contact lens storage solution.

The sterile composition according to the present invention may be prepared according to a process described herein according to any of the respective embodiments.

### Process:

According to an aspect of the invention, there is provided a process for preparing a sterile composition as defined in the present invention comprising an aqueous carrier and liposomes. The process comprises providing an aqueous composition comprising the aqueous carrier and liposomes which comprise at least one bilayer-forming lipid according to any of the respective embodiments described herein and a polymeric compound according to any of the respective embodiments described herein; and subjecting the aqueous composition to a temperature of over 100 °C.

According to an aspect of the invention, the aqueous composition comprising an aqueous carrier and liposomes further comprises an article of manufacture immersed therein according to any of the respective embodiments described herein, such that upon subjecting the aqueous composition to a temperature of over 100 °C, the article of manufacture immersed in the aqueous composition becomes sterile. In some embodiments, the article of manufacture comprises a contact lens. Such a process may allow for efficient and relatively low-cost simultaneous sterilization of solid or semi-solid articles of manufacture and liposome-containing aqueous composition (e.g., which are commonly packaged together), for example, one or more contact lenses immersed in a liposome-containing contact lens solution (e.g., contact lens storage solution).

According to another aspect of the invention, there is provided a process for preparing a sterile article of manufacture having lipids attached to at least a portion of a surface thereof, the process comprising contacting at least a portion of a surface of the article of manufacture with an aqueous composition comprising an aqueous carrier according to any of the respective embodiments described herein and liposomes according to any of the respective embodiments described herein, to thereby obtain an article of manufacture having lipids attached to at least a portion of a surface thereof; and subjecting the article of manufacture having lipids attached to at least a portion of surface thereof to a temperature of over 100 °C. The liposomes comprise at least one bilayer-forming lipid according to any of the respective embodiments described herein and a polymeric compound according to any of the respective embodiments described herein. The sterile article of manufacture is characterized by:
an absence of observable microorganism growth upon testing in fluid thioglycollate medium at a temperature in a range of from 30 to 35 °C for up to 3 days, and/or testing the composition in soybean-casein digest medium at a temperature in a range of from 20 to 25 °C for up to 5 days, and/or when tested for sterility according to U.S. Pharmacopeia General Chapter 71; and/or
a concentration of bacterial endotoxins below a 35 endotoxin units (EU) per ml, as determined using horseshoe crab *(Limulus)* amebocyte lysate according to Chapter 85 of the U.S. Pharmacopeia.

According to an embodiment of the present invention, the article of manufacture comprises a contact lens.

It is to be appreciated that according to this aspect, the lipids attached to at least a portion of the surface may be in a form of liposomes and/or in another form, such as an open bilayer (i.e., a bilayer which does not enclose a volume), e.g., obtainable by "bursting" of the liposomes upon contact with the surface. Optionally, at least a portion of the lipids attached to the surface may optionally be in a different form prior to application of a temperature of over 100 °C than subsequent to application of a temperature of over 100 °C, for example, in a form of liposomes prior to sterilization by heating and in a different form (e.g., an open bilayer) subsequent to sterilization by heating. Alternatively or additionally, the form of the lipid changes gradually (e.g., from liposomes to another form) upon incubation of the article of manufacture in the aqueous composition subsequent to sterilization by heating (e.g., over the course of at least one hour or at least one day, or even at least one month after sterilization).

Such a process may allow for efficient and relatively low-cost sterilization of solid or semi-solid articles of manufacture which has lipids attached to at least a portion of a surface thereof, for example, one or more contact lenses coated with a lipid.

According to an embodiment of the present invention, the temperature to which the aqueous composition is subjected is no more than 150 °C, for example, from 110 °C to 150 °C, or from 115 °C to 150 °C, or from 121 °C to 150 °C, or from 130 °C to 150 °C.

According to an embodiment of the present invention, the temperature to which the aqueous composition is subjected is no more than 140 °C, for example, from 110 °C to 140 °C, or from 115 °C to 140 °C, or from 121 °C to 140 °C, or from 130 °C to 140 °C.

According to an embodiment of the present invention, the temperature to which the aqueous composition is subjected is no more than 134 °C, for example, from 110 °C to 134 °C, or from 115 °C to 134 °C, or from 121 °C to 134 °C.

According to an embodiment of the present invention, the temperature to which the aqueous composition is subjected is no more than 130 °C, for example, from 110 °C to 130 °C, or from 115 °C to 130 °C, or from 121 °C to 130 °C.

According to an embodiment of the present invention, the temperature to which the aqueous composition is subjected is no more than 125 °C, for example, from 110 °C to 125 °C, or from 115 °C to 125 °C, or from 121 °C to 125 °C.

According to an embodiment of the present invention, subjecting the aqueous composition to a temperature of over 100 °C is effected at an elevated pressure, i.e., a pressure greater than ambient atmospheric pressure. Such a pressure may be obtained, for example, by heating the aqueous composition in a closed vessel, such that water vapor formed by the heating participates in elevating the pressure. In some such embodiments, the pressure is such that the boiling point of the aqueous composition at that pressure is equal to the temperature or near (e.g., ±10 °C or ±5 °C) the temperature to which the composition is subjected (according to any of the respective embodiments described herein).

Subjecting the composition to an elevated temperature (and optionally elevated pressure) according to any of the respective embodiments described herein may optionally be effected using a commercial apparatus configured for such use, such as an autoclave.

### Polymeric compound:

According to the present invention, the polymeric compound comprised by a liposome has the general formula I: 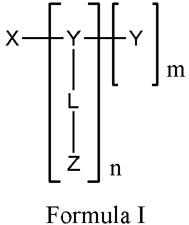 wherein:
m is zero or a positive integer;
n is an integer which is at least 1;
X is a lipid moiety, wherein when X does not comprise a phosphate group, n is at least 2;
Y is a backbone unit which forms a polymeric backbone;
L is absent or is a linking moiety; and
Z has the general formula II: 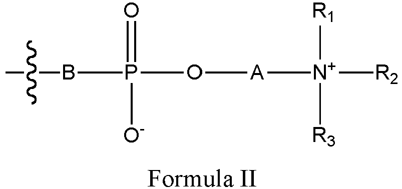
wherein:
   A is a substituted or unsubstituted hydrocarbon;
   B is an oxygen atom or is absent; and
   R₁-R₃ are each independently hydrogen, alkyl, cycloalkyl, heteroalicyclic, aryl or heteroaryl, as described in more detail herein below.

Formula I may also be described herein simply as:

X-[Y(-L-Z)]n[Y]m

which is to be regarded as interchangeable with the schematic depiction hereinabove.

Herein, the term "polymeric" refers a compound having at least 2 repeating units (and more preferably at least 3 repeating units), the repeating units being identical or similar. It is to be appreciated that the compound of general formula I is by definition polymeric when n is at least 2, as it comprises at least 2 of the backbone units represented by Y.

Herein, the phrase "polymeric moiety" refers to the portion of the polymeric compound (according to any of the embodiments described herein relating to general formula I) which has the general formula Ia: 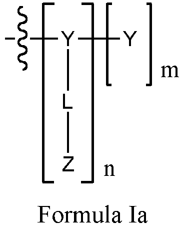 wherein m, n, Y, L and Z are as defined herein for general formula I.

Formula Ia may also be described herein simply as:

[Y(-L-Z)]n[Y]m

which is to be regarded as interchangeable with the schematic depiction hereinabove.

When m is a positive integer, the backbone units [Y(-L-Z)] and [Y] can be arranged to form the polymeric backbone in any order.

Herein, the phrase "polymeric compound" further encompasses compounds having a "polymeric moiety" as described herein having one unit (e.g., according to formula Ia wherein n is 1), provided that the lipid moiety described herein (e.g., the lipid moiety represented by X) has a similar unit. For example, when the lipid moiety comprises a phosphate group (e.g., the lipid moiety is a glycerophospholipid moiety), such that the lipid moiety has a phosphate group and a single unit of the polymeric moiety has a phosphate group, the two phosphate groups may be regarded as repeating units.

In preferred embodiments however, n is at least 2, such that the polymeric moiety *per se* has at least two units. In some embodiments, n is at least 3.

As used herein, the term "backbone unit" refers to a repeating unit, wherein linkage of a plurality of the repeating unit (e.g., sequential linkage) forms a polymeric backbone. A plurality of linked repeating units *per se* is also referred to herein as a "polymeric backbone".

As shown in formulas I and Ia, L and Z together form a pendant group of at least a portion of the backbone units, which group is referred to herein for brevity simply as the "pendant group".

Each backbone unit Y with pendant group (i.e., a unit represented by Y(-L-Z), the number of which is represented by the variable n) and each backbone unit Y without a pendant group (the number of which is represented by the variable m) is also referred to herein as a "monomeric unit".

A backbone unit may optionally be a residue of a polymerizable monomer or polymerizable moiety of a monomer. A wide variety of polymerizable monomers and moieties will be known to the skilled person, and the structure of the residues of such monomers which result upon polymerization (e.g., monomeric units) will also be known to the skilled person.

A "residue of a polymerizable monomer" refers to a modified form of a polymerizable monomer and/or a portion of a polymerizable monomer that remains after polymerization.

A portion of a polymerizable monomer may be formed, for example, by a condensation reaction, e.g., wherein at least one atom or group (e.g., a hydrogen atom or hydroxyl group) in the monomer, and optionally at least two atoms or groups (e.g., a hydrogen atom and a hydroxyl group) in the monomer, is replaced with a covalent bond with another polymerizable monomer.

A modified form of a polymerizable monomer may be formed, for example, by ring-opening (wherein a covalent bond between two atoms in a ring is broken, and the two atoms optionally each become linked to another polymerizable monomer); and/or by adding to an unsaturated bond, wherein an unsaturated bond between two adjacent atoms is broken (e.g., conversion of an unsaturated double bond to a saturated bond, or conversion of an unsaturated triple bond to an unsaturated double bond) and the two atoms optionally each become linked to another polymerizable monomer.

A modified form of a polymerizable monomer may consist essentially of the same atoms as the original monomer, for example, different merely in the rearrangement of covalent bonds, or alternatively, may have a different atomic composition, for example, wherein polymerization includes a condensation reaction (e.g., as described herein).

Examples of backbone units include, without limitation, substituted or unsubstituted hydrocarbons (which may form a substituted or unsubstituted hydrocarbon backbone), such as alkylene units; hydroxycarboxylic acid units (which may form a polyester backbone), e.g., glycolate, lactate, hydroxybutyrate, hydroxyvalerate, hydroxycaproate and hydroxybenzoate units; dicarboxylic acid units (which may form a polyester backbone in combination with a diol and/or a polyamide in combination with a diamine), e.g., adipate, succinate, terephthalate and naphthalene dicarboxylic acid units; diol units (which may form a polyether backbone, or form a polyester backbone in combination with a dicarboxylic acid), e.g., ethylene glycol, 1,2-propanediol, 1,3-propanediol, 1,4-butanediol, and bisphenol A units; diamine units (which may form a polyamide backbone in combination with a dicarboxylic acid), e.g., para-phenylene diamine and alkylene diamines such hexylene diamine; carbamate units (which may form a polyurethane backbone); amino acid residues (which may form a polypeptide backbone); and saccharide residues (which may form a polysaccharide backbone).

According to an embodiment of the present invention, Y is a substituted or unsubstituted alkylene unit.

In some embodiments, Y is a substituted or unsubstituted ethylene unit, that is, an alkylene unit 2 atoms in length.

Polymeric backbones wherein Y is a substituted or unsubstituted ethylene unit may optionally be a polymeric backbone such as formed by polymerizing ethylene (CH₂=CH₂) and/or substituted derivatives thereof (also referred to herein as "vinyl monomers"). Such polymerization is a very well-studied procedure, and one of ordinary skill in the art will be aware of numerous techniques for effecting such polymerization.

It is to be understood that any embodiments described herein relating to a polymeric backbone formed by a polymerization encompass any polymeric backbone having a structure which can be formed by such polymerization, regardless of whether the polymeric backbone was formed in practice by such polymerization (or any other type of polymerization).

As is well known in the art, the unsaturated bond of ethylene and substituted ethylene derivatives becomes saturated upon polymerization, such that the backbone units in a polymeric backbone are saturated, although they may be referred to as units of an unsaturated compound (e.g., a "vinyl monomer" or "olefin monomer") to which they are analogous.

Polymers which can be formed from unsaturated monomers such as vinyl monomers and olefin monomers are also referred to by the terms "polyvinyl" and "polyolefin".

Herein, an "unsubstituted" alkylene unit (e.g., ethylene unit) refers to an alkylene unit which does not have any substituent other than the pendant group discussed herein (represented as (-L-Z)). That is, an alkylene unit attached to the aforementioned pendant group is considered unsubstituted if there are no substituents at any other positions on the alkylene unit.

In some embodiments of any of the respective embodiments described herein, Y has the formula -CR₄R₅-CR₆D-.

When Y is a backbone unit which is not attached to L or Z (i.e., to a pendant group described herein), D is R₇ (an end group, as defined herein); and when Y is a backbone unit which is attached to L or Z, D is a covalent bond or a linking group attaching Y to L or Z. The linking group may optionally be -O-, -S-, arylene, sulfinyl, sulfonyl, phosphate, phosphonyl, phosphinyl, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, or amino.

R₄-R₇ are each independently hydrogen, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, azo, phosphate phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, or amino.

Herein, the phrase "linking group" describes a group (e.g., a substituent) that is attached to two or more moieties in the compound.

Herein, the phrase "end group" describes a group (e.g., a substituent) that is attached to a single moiety in the compound via one atom thereof.

When each of R₄-R₆ is hydrogen, and D is a covalent bond or linking group, Y is an unsubstituted ethylene group attached (via D) to a pendant group described herein.

When each of R₄-R₇ is hydrogen (and D is R₇), Y is an unsubstituted ethylene group which is not attached to a pendant group described herein.

According to an embodiment of the present invention, R₄ and R₅ are each hydrogen. Such embodiments include polymeric backbones formed from many widely used vinyl monomers (including ethylene), including, for example, olefins (e.g., ethylene, propylene, 1-butylene, isobutylene, 4-methyl-1-pentene), vinyl chloride, styrene, vinyl acetate, acrylonitrile, acrylate and derivatives thereof (e.g., acrylate esters, acrylamides), and methacrylate and derivatives thereof (e.g., methacrylate esters, methacrylamides).

According to an embodiment of the present invention, R₆ is hydrogen. In some such embodiments, R₄ and R₅ are each hydrogen.

According to an embodiment of the present invention, R₆ is methyl. Preferably, R₄ and R₅ are each hydrogen. Preferably, the backbone unit is a unit of methacrylate or a derivative thereof (e.g., methacrylate ester, methacrylamide).

According to an embodiment of the present invention, the linking group represented by the variable D is -O-, -C(=O)O-, -C(=O)NH- or phenylene. In exemplary embodiments, D is - C(=O)O-.

For example, the backbone unit may optionally be a vinyl alcohol derivative (e.g., an ester or ether of a vinyl alcohol unit) when D is -O-; an acrylate or methacrylate derivative (e.g., an ester of an acrylate or methacrylate unit) when D is -C(=O)O-; an acrylamide or methacrylamide unit when D is -C(=O)NH-; and/or a styrene derivative (e.g., a substituted styrene unit) when D is phenylene.

According to an embodiment of the present invention, L is a substituted or unsubstituted hydrocarbon from 1 to 10 carbon atoms in length. In some embodiments, the hydrocarbon is unsubstituted. In some embodiments, the hydrocarbon is a linear, unsubstituted hydrocarbon; that is, -(CH₂)ᵢ- wherein i is an integer from 1 to 10.

According to an embodiment of the present invention, L is a substituted or unsubstituted ethylene group. In some embodiments, L is an unsubstituted ethylene group (-CH₂CH₂-).

According to an embodiment of the present invention, B is an oxygen atom. In some such embodiments, L is a hydrocarbon according to any of the respective embodiments described herein (i.e., L is not absent), and Z is a phosphate group attached to L.

According to an embodiment of the present invention, B is absent. Preferably, L is a hydrocarbon according to any of the respective embodiments described herein (i.e., L is not absent), and Z is a phosphonate group attached to L. Preferably, L is also absent, such that the phosphorus atom of formula II is attached directly to Y.

According to an embodiment of the present invention, A is a substituted or unsubstituted hydrocarbon from 1 to 4 carbon atoms in length.

According to an embodiment of the present invention, A is an unsubstituted hydrocarbon. In some such embodiments, the unsubstituted hydrocarbon is from 1 to 4 carbon atoms in length. In some embodiments, the hydrocarbon is a linear, unsubstituted hydrocarbon; that is, -(CH₂)ⱼ-wherein j is an integer from 1 to 4.

According to an embodiment of the present invention, A is a substituted or unsubstituted ethylene group.

According to an embodiment of the present invention, A is an unsubstituted ethylene group (-CH₂CH₂-). In such embodiments, the moiety having general formula II (represented by the variable Z) is similar or identical to a phosphoethanolamine or phosphocholine moiety. Phosphoethanolamine and phosphocholine moieties are present in many naturally occurring compounds (e.g., phosphatidylcholines, phosphatidylethanolamines).

According to an embodiment of the present invention, A is an ethylene group substituted by a C-carboxy group. In some embodiments, the C-carboxy is attached to the carbon atom adjacent to the nitrogen atom depicted in formula II (rather than the carbon atom attached to the depicted oxygen atom). In such embodiments, the moiety having general formula II (represented by the variable Z) is similar or identical to a phosphoserine moiety. Phosphoserine is present in many naturally occurring compounds (e.g., phosphatidylserines).

Without being bound by any particular theory, it is believed that moieties similar or identical to naturally occurring moieties such as phosphocholine, phosphoethanolamine and/or phosphoserine may be particularly biocompatible.

According to an embodiment of the present invention, R₁-R₃ (the substituents of the nitrogen atom depicted in general formula II) are each independently hydrogen or C₁₋₄-alkyl. In some embodiments, R₁-R₃ are each independently hydrogen or methyl. In some embodiments, R₁-R₃ are each methyl. In some such embodiments, R₁-R₃ are each hydrogen.

The variable n may be regarded as representing a number of backbone units (represented by the variable Y) which are substituted by the pendant group represented by (-L-Z), and the variable m may be regarded as representing a number of backbone units which are not substituted by such a pendant group. The sum n+m may be regarded as representing the total number of backbone units in the polymeric backbone. The ratio n/(n+m) may be regarded as representing the fraction of backbone units which are substituted by the pendant group represented by (-L-Z).

The backbone unit Y substituted by the pendant group may be the same as or different than the backbone unit Y which is not substituted by the pendant group (e.g., when m is at least 1).

The plurality (indicated by the variable n) of backbone units Y substituted by the pendant group may be the same as each other or different from each other.

In addition, the plurality (indicated by the variable n) of pendant groups (-L-Z) attached to a plurality of backbone units Y may be the same as each other or different from each other (e.g., may differ in the identity of any one or more of A, B, R₁, R₂, R₃ and L).

According to an embodiment of the present invention wherein more than one backbone unit Y is not substituted by the pendant group described herein (i.e., when m is more than 1), the plurality (indicated by the variable m) of backbone units Y which are substituted by the pendant group may be the same as each other or different from each other.

The number of types of backbone units substituted by the pendant group, the number of types of backbone units not substituted by the pendant group (if any such units are present), and/or the number of types of pendant group in the polymeric moiety may each independently be any number (e.g., 1, 2, 3, 4, 5, 6, 7, 8, 9, 10 or more).

According to an embodiment of the present invention, the polymeric moiety is a copolymer moiety, that is, the polymeric moiety comprises at least two different types of monomeric unit. The different types of monomeric unit may differ in whether they comprise the pendant group (-L-Z) according to any of the respective embodiments described herein (e.g., when m is at least 1), differ in the type of backbone unit Y, and/or differ in the type of pendant group (-L-Z).

In some embodiments of the present invention the backbone unit Y in each of the Y(-L-Z) units may optionally be the same or different, while the L and Z moieties are the same among the Y(-L-Z) units. In some such embodiments, backbone units not substituted by the pendant group (if any such units are present) may optionally be the same as backbone unit Y in each of the Y(-L-Z) units. Alternatively, backbone units not substituted by the pendant group (if any such units are present) may optionally be different than backbone unit Y in each of the Y(-L-Z) units (while optionally being the same among all backbone units not substituted by the pendant group).

In some embodiments the present invention, the L moiety in each of the Y(-L-Z) units may optionally be the same or different, while the backbone units Y and the Z moieties are the same among the Y(-L-Z) units. In some such embodiments, backbone units not substituted by the pendant group (if any such units are present) may optionally be the same as backbone unit Y in each of the Y(-L-Z) units. Alternatively, backbone units not substituted by the pendant group (if any such units are present) may optionally be different than backbone unit Y in each of the Y(-L-Z) units (while optionally being the same among all backbone units not substituted by the pendant group).

In some embodiments of the present invention, the Z moiety in each of the Y(-L-Z) units may optionally be the same or different, while the backbone units Y and the Z moieties are the same among the Y(-L-Z) units. In some such embodiments, backbone units not substituted by the pendant group (if any such units are present) may optionally be the same as backbone unit Y in each of the Y(-L-Z) units. Alternatively, backbone units not substituted by the pendant group (if any such units are present) may optionally be different than backbone unit Y in each of the Y(-L-Z) units (while optionally being the same among all backbone units not substituted by the pendant group).

According to the present invention wherein the polymeric moiety is a copolymer moiety, any two or more different types of monomeric unit may be distributed randomly or non-randomly throughout the polymeric moiety. When different types of monomeric unit are distributed non-randomly, the copolymer may be one characterized by any non-random distribution, for example, an alternating copolymer, a periodic copolymer, and/or a block copolymer.

According to the present invention , at least a portion of the monomeric units of the polymeric moiety comprise a targeting moiety (according to any of the embodiments described herein relating to a targeting moiety). In some embodiments, the targeting moiety, monomeric unit comprising a targeting moiety and/or polymeric compound comprising a targeting moiety may optionally be, respectively, any targeting moiety, monomeric unit comprising a targeting moiety and/or polymeric compound comprising a targeting moiety described in International Patent Application publication WO 2018/150429.

Herein, a "targeting moiety" refers to a moiety which is capable of bringing a compound (e.g., a compound according to some embodiments of the invention) into proximity with a selected substance and/or material (which is referred to herein as a "target"). The target is optionally a cell (e.g., a proliferating cell associated with the proliferative disease or disorder), wherein the proximity is such that the targeting moiety facilitates attachment and/or internalization of the compound into a target cell, and such that the compound may exert a therapeutic effect.

A targeting moiety may optionally be comprised by a backbone unit Y according to any of the respective embodiments described herein, linking moiety L according to any of the respective embodiments described herein, and/or moiety Z according to any of the respective embodiments described herein, for example, wherein a substituent according to any of the respective embodiments described herein comprises (and optionally consists of) the targeting moiety. For example, in some embodiments wherein at least a portion of backbone units Y have the formula - CR₄R₅-CR₆D- (as described herein in any of the respective embodiments), any one or more of R₄-R₆ and D (optionally wherein D is R₇ as described herein) comprises a targeting moiety according to any of the respective embodiments described herein (e.g., wherein any one or more of R₄-R₆ and D is a substituted group, comprising a substituent which is a targeting moiety), and optionally any one or more R₄-R₆ and D is a targeting moiety. However, many other structures of monomeric units comprising a substituent which comprises (and optionally consist of) a targeting moiety are also encompassed by embodiments of the invention.

When Y is a backbone unit which is not attached to L or Z (i.e., to a pendant group as described herein), D is R₇ (an end group, as defined herein); and when Y is a backbone unit which is attached to L or Z, D is a covalent bond or a linking group attaching Y to L or Z. The linking group may optionally be -O-, -S-, arylene, sulfinyl, sulfonyl, phosphate, phosphonyl, phosphinyl, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, or amino.

R₄-R₇ are each independently hydrogen, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, azo, phosphate phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, or amino.

According to an embodiment of the present invention, the polymeric moiety is a copolymer moiety wherein at least one type of a monomeric unit comprises a targeting moiety (according to any of the respective embodiments described herein) and at least one type of a monomeric unit does not comprise such a targeting moiety. The distribution of a monomeric unit comprising a targeting moiety may be in accordance with any distribution described herein of a monomeric unit in a copolymer moiety (e.g., random, alternating, periodic copolymer, and/or block copolymer).

According to an embodiment of the present invention wherein a portion of monomeric units comprise a targeting moiety, the monomeric units comprising a targeting moiety are, on average, closer to a terminus of the polymeric moiety distal to the lipid moiety, e.g., an average distance (as measured in atoms or backbone units along the backbone of the polymeric moiety) of monomeric units comprising a targeting moiety from the lipid moiety is greater than an average distance of the other monomeric units from the lipid moiety.

According to an embodiment of the present invention, at least a portion (and optionally all) of the monomeric units comprising a targeting moiety form a block (of one or more monomeric units) near (and optionally at) a terminus of the polymeric moiety distal to the lipid moiety. In some such embodiments, the copolymer moiety contains a single monomeric unit which comprises a targeting moiety, and the monomeric unit is at a terminus of the polymeric moiety distal to the lipid moiety.

Without being bound by any particular theory, it is assumed that a targeting moiety located distal to the lipid moiety may be more effective as a targeting moiety (e.g., more effective at binding to a target), for example, due to the targeting moiety being less sterically shielded (e.g., by a surface to which the lipid moiety is associated) and therefore more exposed to and thus better able to make contact with targets in an aqueous environment.

According to an embodiment of the present invention wherein m is at least 1, the polymeric moiety comprises a monomeric unit which comprises a targeting moiety, and the monomeric unit is at a terminus of the polymeric moiety distal to the lipid moiety. In such embodiments, the compound represented by general formula I has the formula Ib: 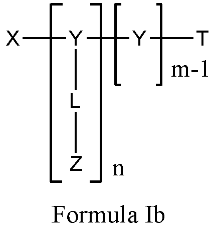 wherein:
T is a monomeric unit comprising a targeting moiety (according to any of the respective embodiments described herein);
X and T are attached to distal termini of the moiety represented by [Y(-L-Z)]n[Y]m-1; and
X, Y, L, Z, n and m are defined in accordance with any of the embodiments described herein relating to general formula I, with the proviso that m is at least 1.

It is to be understood that T in formula Ib is a type of monomeric unit represented by Y (i.e., without the pendant group represented by (-L-Z)) in formulas I and Ia, and the number of monomeric units represented by Y (i.e., without the pendant group represented by (-L-Z)) other than T is represented by the value m-1, such that the total number of monomeric units without the pendant group represented by (-L-Z)), including T, is represented by the variable m, as in formulas I and Ia.

In some embodiments, m is 1, such that m-1 is zero, and the compound represented by formula Ib consequently has the formula: X-[Y(-L-Z)]n-T, wherein L, T, X, Y, Z and n are defined in accordance with any of the embodiments described herein.

A monomeric unit comprising a targeting moiety according to any of the respective embodiments described herein may optionally be prepared by preparing a monomer comprising a targeting moiety, and using the monomer to prepare a polymeric moiety described herein (e.g., by polymerization of monomers according to any of the respective embodiments described herein) and/or by modifying a monomeric unit in a polymeric moiety subsequently to preparation of a polymeric moiety (e.g., by polymerization of monomers according to any of the respective embodiments described herein), using any suitable technique known in the art, including, but not limited to, techniques for conjugation.

According to an embodiment of the present invention, the polymeric moiety does not comprise a targeting moiety described herein according to any of the respective embodiments.

According to the present invention, the percentage of backbone units (represented by the variable Y) which are substituted by the pendant group represented by (-L-Z) (as represented by the formula 100%*n/(n+m)) is at least 20 %. In some embodiments, the percentage of backbone units substituted by the aforementioned pendant group is at least 30 %. In some embodiments, the percentage of backbone units substituted by the aforementioned pendant group is at least 40 %. In some embodiments, the percentage of backbone units substituted by the aforementioned pendant group is at least 50 %. In some embodiments, the percentage of backbone units substituted by the aforementioned pendant group is at least 60 %. In some embodiments, the percentage of backbone units substituted by the aforementioned pendant group is at least 70 %. In some embodiments, the percentage of backbone units substituted by the aforementioned pendant group is at least 80 %. In some embodiments, the percentage of backbone units substituted by the aforementioned pendant group is at least 90 %. In some embodiments, the percentage of backbone units substituted by the aforementioned pendant group is at least 95 %. In some embodiments, the percentage of backbone units substituted by the aforementioned pendant group is at least 98 %.

According to the present invention, m is 0, such that each of the backbone units (represented by the variable Y) is substituted by the pendant group represented by (-L-Z).

According to the present invention, n is at least 5. In some embodiments, n is at least 10. In some embodiments, n is at least 15.

According to the present invention , n is in a range of from 2 to 1,000, including any intermediate values and subranges therebetween. In some embodiments of any of the embodiments described herein, n is in a range of from 2 to 500, including any intermediate values and subranges therebetween. In some embodiments of any of the embodiments described herein, n is in a range of from 2 to 200, including any intermediate values and subranges therebetween. In some embodiments of any of the embodiments described herein, n is in a range of from 2 to 100, including any intermediate values and subranges therebetween. In some embodiments of any of the embodiments described herein, n is in a range of from 2 to 50, including any intermediate values and subranges therebetween. In some embodiments of any of the embodiments described herein, n is in a range of from 10 to 100, including any intermediate values and subranges therebetween. In some embodiments of any of the embodiments described herein, n is in a range of from 10 to 50, including any intermediate values and subranges therebetween. In some embodiments of any of the embodiments described herein, n is in a range of from 50 to 100, including any intermediate values and subranges therebetween. In some embodiments of any of the embodiments described herein, n is in a range of from 30 to 100, including any intermediate values and subranges therebetween. In some embodiments of any of the embodiments described herein, n is in a range of from 20 to 120, including any intermediate values and subranges therebetween. In some such embodiments, m is 0.

According to the present invention, n is in a range of from 3 to 1,000, including any intermediate values and subranges therebetween. According to the present invention, n is in a range of from 3 to 500, including any intermediate values and subranges therebetween. According to the present invention, n is in a range of from 3 to 200, including any intermediate values and subranges therebetween. According to the present invention, n is in a range of from 3 to 100, including any intermediate values and subranges therebetween. According to the present invention, n is in a range of from 3 to 50, including any intermediate values and subranges therebetween. According to the present invention, n is in a range of from 5 to 50, including any intermediate values and subranges therebetween. According to the present invention, n is in a range of from 10 to 50, including any intermediate values and subranges therebetween. According to the present invention, n is in a range of from 10 to 25, including any intermediate values and subranges therebetween. In some such embodiments, m is 0.

According to the present invention, m is in a range of from 0 to 1,000, including any intermediate values and subranges therebetween. Preferably, n is in a range of from 2 to 1,000, such that the total number of backbone units is in a range of from 2 to 2,000. More preferably, n is in a range of from 3 to 1,000. Preferably, n is in a range of from 3 to 500. Preferentially, n is in a range of from 3 to 200. Preferably, n is in a range of from 3 to 100. In some embodiments, n is in a range of from 3 to 50 Preferably, n is in a range of from 5 to 50. Preferably, n is in a range of from 10 to 50.

According to the present invention, m is in a range of from 0 to 500, including any intermediate values and subranges therebetween. Preferably, n is in a range of from 2 to 1,000. In some such embodiments, n is in a range of from 3 to 1,000. Preferably, n is in a range of from 3 to 500. Preferably, n is in a range of from 3 to 200. Preferably, n is in a range of from 3 to 100. In some embodiments, n is in a range of from 10 to 100. Preferably, n is in a range of from 30 to 100. Preferably, n is in a range of from 50 to 100. In some embodiments, n is in a range of from 3 to 50. Preferably, n is in a range of from 10 to 50. Preferably, n is in a range of from 5 to 50. Preferably, n is in a range of from 10 to 50.

According to the present invention, m is in a range of from 0 to 200, including any intermediate values and subranges therebetween. Preferably, n is in a range of from 2 to 1,000. Preferably, n is in a range of from 3 to 1,000. Preferably, n is in a range of from 3 to 500. Preferably, n is in a range of from 3 to 200. Preferably, n is in a range of from 3 to 100. Preferably, n is in a range of from 10 to 100. Preferably, n is in a range of from 30 to 100. Preferably, n is in a range of from 50 to 100. Preferably, n is in a range of from 3 to 50. Preferably, n is in a range of from 10 to 50. Preferably, n is in a range of from 3 to 50. Preferably, n is in a range of from 10 to 50. Preferably, n is in a range of from 5 to 50. Preferably, n is in a range of from 10 to 50.

According to the present invention, m is in a range of from 0 to 100, including any intermediate values and subranges therebetween Preferably, n is in a range of from 2 to 1,000. Preferably, n is in a range of from 3 to 1,000. Preferably, n is in a range of from 3 to 500. Preferably, n is in a range of from 3 to 200. Preferably, n is in a range of from 3 to 100. Preferably, n is in a range of from 10 to 100. Preferably, n is in a range of from 30 to 100. Preferably, n is in a range of from 50 to 100. Preferably, n is in a range of from 3 to 50. Preferably, n is in a range of from 10 to 50. Preferably, n is in a range of from 10 to 50. Preferably, n is in a range of from 3 to 50. Preferably, n is in a range of from 5 to 50. Preferably, n is in a range of from 10 to 50.

According to the present invention, m is in a range of from 0 to 50, including any intermediate values and subranges therebetween. Preferably, n is in a range of from 2 to 1,000. Preferably, n is in a range of from 3 to 1,000 Preferably, n is in a range of from 3 to 500. Preferably, n is in a range of from 3 to 200. Preferably, n is in a range of from 3 to 100. Preferably, n is in a range of from 10 to 100. Preferably, n is in a range of from 30 to 100. Preferably, n is in a range of from 50 to 100. Preferably, n is in a range of from 3 to 50. Preferably, n is in a range of from 10 to 50. Preferably, n is in a range of from 10 to 50. Preferably, n is in a range of from 3 to 50. Preferably, n is in a range of from 5 to 50. Preferably, n is in a range of from 10 to 50.

According to the present invention, m is in a range of from 0 to 20, including any intermediate values and subranges therebetween. Preferably, n is in a range of from 2 to 1,000. Preferably, n is in a range of from 3 to 1,000. In some embodiments, n is in a range of from 3 to 500. Preferably, n is in a range of from 3 to 200. Preferably, n is in a range of from 3 to 100. Preferably, n is in a range of from 10 to 100. Preferably, n is in a range of from 30 to 100. Preferably, n is in a range of from 50 to 100 Preferably, n is in a range of from 3 to 50. In some embodiments, n is in a range of from 10 to 50. Preferably, n is in a range of from 10 to 50. Preferably, n is in a range of from 3 to 50. Preferably, n is in a range of from 5 to 50. Preferably, n is in a range of from 10 to 50.

According to the present invention, m is in a range of from 0 to 10, including any intermediate values and subranges therebetween. Preferably, n is in a range of from 2 to 1,000. Preferably, n is in a range of from 3 to 1,000. Preferably, n is in a range of from 3 to 500. Preferably, n is in a range of from 3 to 200. Preferably, n is in a range of from 3 to 100. Preferably, n is in a range of from 10 to 100. Preferably, n is in a range of from 30 to 100. Preferably, n is in a range of from 50 to 100. Preferably, n is in a range of from 3 to 50. Preferably, n is in a range of from 10 to 50. Preferably, n is in a range of from 10 to 50. Preferably, n is in a range of from 3 to 50. Preferably, n is in a range of from 5 to 50. Preferably, n is in a range of from 10 to 50.

According to the present invention, a lipid moiety (represented by the variable X in formula I herein) is attached to a polymeric moiety according to any of the embodiments described herein relating to the polymeric moiety.

The lipid moiety may optionally be derived from any lipid known in the art (including, but not limited to, a naturally occurring lipid). Derivation of the lipid moiety from the lipid may optionally consist of substituting a hydrogen atom at any position of the lipid with the polymeric moiety represented in general formula I by [Y(-L-Z)]n[Y]m (i.e., the polymeric moiety represented by general formula Ia).

According to the present invention, a polymeric moiety in the liposome comprises a lipid moiety represented by the variable X in formula I, according to any of the respective embodiments described herein which comprises a residue of a bilayer-forming lipid (e.g., a glycerophospholipid) which is comprised by the liposome in addition to the polymeric moiety according to any of the respective embodiments described herein, or which is closely related to such a bilayer-forming lipid comprised by the liposome; for example, wherein the lipid moiety comprises a glycerophospholipid residue and the liposome comprises another glycerophospholipid as a bilayer-forming lipid (e.g., optionally wherein fatty acid residues in the glycerophospholipid residue have about the same length as fatty acid residues in the bilayer-forming lipid, and optionally wherein the fatty acid residues in the glycerophospholipid residue are substantially the same as the fatty acid residues in the bilayer-forming lipid).

Without being bound by any particular theory, it is believed that similarity between a lipid moiety of a polymeric moiety and a bilayer-forming lipid facilitates anchorage of the lipid moiety of the polymeric moiety in a liposome comprising the bilayer-forming lipid.

According to the present invention, the lipid moiety according to any of the respective embodiments described herein is attached to a Y(-L-Z) unit according to any of the embodiments described herein relating to Y, L and/or Z, that is, backbone unit substituted by the pendant group described herein rather than a backbone unit not substituted by the pendant group.

According to the present invention, the lipid moiety is a moiety of a lipid which is a fatty acid, a monoglyceride, a diglyceride, a triglyceride, a glycerophospholipid, a sphingolipid, or a sterol. **In** some embodiments, the lipid is a glycerophospholipid, for example, a phosphatidyl ethanolamine, a phosphatidyl serine, a phosphatidyl glycerol and/or a phosphatidyl inositol.

According to the present invention, the lipid moiety comprises at least one fatty acid moiety (e.g., an acyl group derived from a fatty acid). The fatty acid moiety may be derived from a saturated or unsaturated fatty acid. For example, the lipid moiety may consist of a fatty acid moiety, or be a monoglyceride moiety comprising one fatty acid moiety, a diglyceride moiety comprising two fatty acid moieties, or a triglyceride moiety comprising three fatty acid moieties.

Examples of fatty acid moieties which may optionally be comprised by the lipid moiety include, without limitation, lauroyl, myristoyl, palmitoyl, stearoyl, palmitoleoyl, oleoyl, and linoleoyl.

According to the present invention, the lipid moiety represented by the variable X has the general formula **III:** 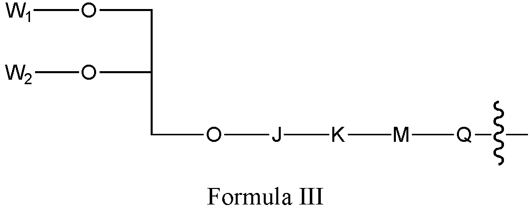
W₁ and W₂ are each independently hydrogen, alkyl, alkenyl, alkynyl or acyl, wherein at least one of W₁ and W₂ is not hydrogen;
J is -P(=O)(OH)-O-, or J is absent (such that K is attached directly to the depicted oxygen atom of a glycerol moiety);
K is a substituted or unsubstituted hydrocarbon from 1 to 10 carbon atoms in length, or K is absent (such that M is attached directly to J or, when J is absent, M is attached directly to the depicted oxygen atom of a glycerol moiety);
M is a linking group which is -O-, -S-, amino, sulfinyl, sulfonyl, phosphate, phosphonyl, phosphinyl, carbonyl, thiocarbonyl, urea, thiourea, carbamyl, thiocarbamyl, amido, carboxy, or sulfonamide, or M is absent; and
Q is a substituted or unsubstituted hydrocarbon from 1 to 10 carbon atoms in length, and is attached to a backbone unit of the polymeric backbone according to any of the respective embodiments described herein,
wherein when M is absent, K is also absent.

When M is absent, K is also absent, and Q is attached directly to J or, when J is absent, Q is attached directly to the depicted oxygen atom of a glycerol moiety.

According to an aspect of the present invention, one of W₁ and W₂ is hydrogen and the other is not hydrogen.

According to an aspect of the present invention , neither W₁ nor W₂ is hydrogen.

According to an aspect of the present invention , at least one of W₁ and W₂ is an alkyl, alkenyl, alkynyl or acyl, which is from 10 to 30 carbon atoms in length. In some embodiments, each of W₁ and W₂ is from 10 to 30 carbon atoms in length.

Examples of acyl groups which may optionally serve independently as W₁ and/or W₂ include, without limitation, lauroyl, myristoyl, palmitoyl, stearoyl, palmitoleoyl, oleoyl, and linoleoyl.

According to an aspect of the present invention, J is -P(=O)(OH)-O- (e.g., the lipid moiety is a glycerophospholipid).

Herein, the length of the hydrocarbon represented by the variable K refers to the number of atoms separating J and M (i.e., along the shortest path between J and M) as depicted in formula III.

When K is a substituted hydrocarbon, M may be attached to a carbon atom of the hydrocarbon *per se,* or be attached to a substituent of the hydrocarbon.

According to an aspect of the present invention, K is an ethanolamine moiety (e.g., -CH₂-CH₂-NH-, or -CH₂-CH₂- attached to a nitrogen atom), a serine moiety (e.g., -CH₂-CH(CO₂H)-NH-, or -CH₂-CH(CO₂H)- attached to a nitrogen atom), a glycerol moiety (e.g., -CH(OH)-CH(OH)-CH-O-) and an inositol moiety (e.g., -cyclohexyl(OH)₄-O-). In some embodiments, J is -P(=O)(OH)-O-.

According to an aspect of the present invention, M is amido, optionally -C(=O)NH-.

According to an aspect of the present invention, the nitrogen atom of the amido is attached to K. In some such embodiments, K is an ethanolamine or serine moiety described herein.

According to an aspect of the present invention, J is absent (e.g., wherein the lipid moiety is a glycerolipid moiety which is not a glycerophospholipid moiety). Preferably, K is also absent, such that M is attached directly to the depicted oxygen atom of a glycerol moiety or, when M is also absent, Q is attached directly to the depicted oxygen atom of a glycerol moiety (e.g., wherein the glycerolipid is a monoacylglycerol derivative or diacylglycerol derivative). In some embodiments, M is a carbonyl linking group, such that attachment of M to the aforementioned oxygen atom of the glycerol moiety is via an ester bond.

According to an aspect of the present invention, Q is a substituted or unsubstituted methylene group. In some such embodiments, M comprises a carbonyl (i.e., C(=O)) linking group. In some embodiments, M is amido (which comprises a carbonyl and a nitrogen atom. In some embodiments, the C(=O) (e.g., of the amido) is attached to Q. In some embodiments, M consists of a carbonyl linking group.

According to an aspect of the present invention, Q is a methylene group substituted by one or two substituents. In some embodiments, the methylene group is substituted by one or two alkyl groups (e.g., C₁₋₄-alkyl).

According to an aspect of the present invention, Q is a methylene group substituted by two substituents. In some embodiments, the methylene group is substituted by two alkyl groups (e.g., C₁₋₄-alkyl). In some embodiments, the alkyl groups are methyl, such that Q is dimethylmethylene (-C(CH₃)₂-).

As exemplified in the Examples section herein, a substituted methylene (e.g., di-substituted methylene) represented by the variable Q is particularly suitable for participating in polymerization reactions (e.g., as an initiator), because a free radical and/or ion on the methylene may be stabilized by the substituent(s) thereof.

As further exemplified herein, formation of an amido group (represented by the variable M) may serve as a convenient way to attach the abovementioned substituted methylene to a lipid (e.g., a naturally occurring lipid) such as a phosphatidylethanolamine or phosphatidylserine.

As further exemplified herein, formation of an ester bond between a carbonyl (e.g., comprised by M) and an oxygen atom of a lipid (e.g., an oxygen atom of a glycerol moiety) may serve as a convenient way to attach the abovementioned substituted methylene to a lipid (e.g., a naturally occurring lipid) such as a monoacylglycerol, diacylglycerol, phosphatidyl glycerol or phosphatidyl inositol.

### Article of manufacture:

As discussed herein, the sterile composition according to the present invention may optionally comprise an article of manufacture immersed therein; and a sterile article of manufacture may optionally be obtained (e.g., immersed in a liposome-containing composition and/or having lipids attached to at least a portion of a surface thereof) according to a respective process described herein.

According to an aspect of the present invention, there is provided a sterile article of manufacture prepared according to a process according to any of the embodiments described herein relating to a process for obtaining a sterile article of manufacture.

Herein, the term "article of manufacture" refers to any article produced from materials in a manner which results in new forms, qualities, properties or combinations of the materials, and which does not include a portion of a human being. It is to be understood that this definition is not necessarily identical with a standard legal definition of the term.

The article of manufacture, according to any of the respective embodiments described herein, preferably comprises one or more substances in a form which does not exist in nature. The form which does not exist in nature may optionally comprise natural substances in a combination which does not exist in nature, and/or may optionally comprise one or more substances which do not occur in nature.

According to an aspect of the present invention, the article of manufacture has lipids non-covalently attached to at least a portion of a surface thereof, for example, wherein at least a portion of the lipids are in a form of a lipid bilayer (i.e., two parallel layers of molecules of the lipid). **In** some such embodiments, at least a portion of the molecules of the lipid (e.g., in a bilayer) are oriented such that polar groups thereof (e.g., charged groups) face outwards at a surface of the article of manufacture.

As used herein, the phrase "face outwards at a surface" refers to a group in a molecule (e.g., a lipid) at a surface of a substrate (e.g., a surface of an article of manufacture to which a lipid is attached) which is closer to an external environment than the center of mass of the molecule is to the external environment, and farther from the substrate than the center of mass of the molecule is from the substrate.

Without being bound by any particular theory, it is believed that outwards facing polar groups (e.g., charged groups) according to some embodiments of the invention are effect highly effective lubrication and/or inhibition of adhesion, biofouling and/or biofilm formation due, at least in part, to properties of hydrated polar groups (e.g., hydration lubrication), especially hydrated charged groups.

According to the present invention herein, the portion of the article of manufacture to which the lipids are attached may comprise any type of material or combination of different types of material, including inorganic material and/or organic material, in crystalline, amorphous and/or gel (e.g., hydrogel) forms, for example, metal, mineral, ceramic, glass, polymer (e.g., synthetic polymer, biopolymer), plant and/or animal biomass (e.g., wood or leather), and combinations thereof.

According to an aspect of the present invention, the article of manufacture is a medical device, e.g., a medical device having lipids attached to at least a portion of a surface thereof. In some embodiments, the medical device is a device designed to come into contact with a part of the body susceptible to infection, such as an internal portion of the body, a mucous membrane and/or a surface of the eye. Examples of such medical devices include, without limitation, surgical tools and implants (which are for coming into contact with an internal portion of the body) and contact lenses (which are for contacting a surface of the eye).

As used herein throughout, the phrase "medical device" includes any material or device that is used on, in, or through a subject's body, for example, in the course of medical treatment (e.g., for a disease or injury). The subject may be human or a non-human animal, such that the phrase "medical device" encompasses veterinary devices. Medical devices include, but are not limited to, such items as medical implants (including permanent implants and transient implants), wound care devices, medical devices for drug delivery, contact lenses and body cavity and personal protection devices. The medical implants include, but are not limited to, catheters (e.g., urinary catheters, intravascular catheters), injection ports, intubation equipment, dialysis shunts, wound drain tubes, skin sutures, vascular grafts, implantable meshes, intraocular devices, heart valves, and the like. Wound care devices include, but are not limited to, general wound dressings, biologic graft materials, tape closures and dressings, and surgical incise drapes. Medical devices for drug delivery include, but are not limited to, needles, drug delivery skin patches, drug delivery mucosal patches and medical sponges. Body cavity and personal protection devices, include, but are not limited to, tampons, sponges, surgical and examination gloves, and toothbrushes. Birth control devices include, but are not limited to, intrauterine devices (IUDs), diaphragms and condoms.

In the context of medical devices, it is to be understood that the macroscopic medical device is coated by a bilayer described herein.

Examples of suitable articles of manufacture include, without limitation:
medical devices (e.g., contact lenses, pacemakers, heart valves, replacement joints, catheters, catheter access ports, dialysis tubing, gastric bands, shunts, screw plates, artificial spinal disc replacements, internal implantable defibrillators, cardiac resynchronization therapy devices, implantable cardiac monitors, mitral valve ring repair devices, left ventricular assist devices (LVADs), artificial hearts, implantable infusion pumps, implantable insulin pumps, stents, implantable neurostimulators, maxillofacial implants, dental implants, and the like);
packages or containers, for example, packages or containers for food and/or beverages (e.g., packages for meat and/or dairy products and/or containers for storage or transportation of meat and/or dairy products, such as storage tanks, raw milk holding equipment, dairy processing operations conveyer belts, tube walls, gaskets, rubber seals, stainless steel coupons, piping systems, filling machine, silo tanks, heat exchangers, post-pasteurization equipment, pumps, valves, separators, and spray devices), medical device packages, agricultural packages and containers (of agrochemicals), blood sample or other biological sample packages and containers, and any other packages or containers of various articles; and
elements of water treatment systems (e.g., for containing and/or transporting and/or treating aqueous media or water), devices, containers, filters, tubes, solutions and gases and the like.

According to an aspect of the present invention, the article of manufacture comprises a hydrogel surface, e.g., having lipids attached to at least a portion thereof.

Contact lenses are an exemplary article of manufacture comprising a hydrogel surface. In some embodiments, the contact lens comprises a hydrogel surface and a rigid center. In some embodiments, the contact lens consists essentially of a hydrogel.

The hydrogel may comprise any material known in the art for use in contact lens hydrogels. Examples of such hydrogel materials include, without limitation, alphafilcon A, asmofilcon A, balafilcon A, bufilcon A, comfilcon A, crofilcon, deltafilcon A, dimefilcon, droxifilcon A, enfilcon A, etafilcon A, galyfilcon A, hefilcon A, hefilcon B, hilafilcon A, hilafilcon B, hioxifilcon A, hioxifilcon D, isofilcon, lidofilcon A, lidofilcon B, lotrafilcon B, mafilcon, methafilcon A, methafilcon B, narafilcon A, narafilcon B, ocufilcon A, ocufilcon B, ofilcon A, omafilcon A, perfilcon, phemfilcon A, polymacon, scafilcon A, senofilcon A, surfilcon, tefilcon, tetrafilcon A, tetrafilcon B, vifilcon A, and xylofilcon A.

According to an aspect of the present invention, the hydrogel comprises a polymer consisting of poly(2-hydroxyethyl methacrylate) and/or a silicone. In some embodiments, the polymer comprises a silicone. Such polymers may optionally comprise small amounts of additional monomers (e.g., cross-linking monomers) copolymerized with the 2-hydroxyethyl methacrylate or silicone monomer. For example, 2-hydroxyethyl methacrylate may optionally be copolymerized with vinyl pyrrolidone, methyl methacrylate, methacrylic acid (an anionic monomer), ethylene glycol dimethacrylate (a cross-linking monomer) and/or 3-(ethyldimethyl-ammonium)propyl methacrylamide (a cationic monomer) in a contact lens hydrogel.

### Uses of the composition:

In view of their sterile nature, compositions according to any of the embodiments described herein may be useful for use in a physiological environment, such as internal physiological environment or an ophthalmic environment. Accordingly, the aqueous carrier according to any of the respective embodiments described herein may optionally be selected in accordance with an intended use, for example, a physiologically acceptable carrier and/or an ophthalmically acceptable carrier.

Herein throughout, the term "physiologically acceptable carrier" refers to a carrier or a diluent that does not cause significant irritation to a subject upon administration in the intended manner, and does not abrogate the activity and properties of the composition (e.g., the ability of liposomes therein to treat a condition and/or reduce a friction coefficient of a surface, as described herein in any one of the respective embodiments). Examples, without limitations, of suitable aqueous carriers include saline, and emulsions and/or mixtures of organic solvents with water (or saline).

Herein, the phrase "ophthalmically acceptable carrier" refers to a carrier or a diluent that does not cause significant irritation to a subject when contacted with an eye (e.g., cornea and/or sclera) of the subject, and does not abrogate the activity and properties of the composition (e.g., the ability of liposomes therein to reduce a friction coefficient of a surface of a contact lens and/or a surface of the eye).

A use in a physiological environment may optionally be for reducing a friction coefficient (also referred to herein as "lubrication", "lubricating" and variants thereof) of a physiological surface (e.g., an articular surface or a surface of an eye) and/or non-physiological surface (e.g., contact lens surface), for example, in treating a disease or disorder associated with an increased friction coefficient of the surface. Reduction of a friction coefficient of a surface may optionally be effected by any one or more compound present in liposomes (according to any of the respective embodiments described herein), including a bilayer-forming lipid and/or polymeric compound according to any of the respective embodiments described herein.

In an embodiment of the present invention, the sterile composition is for rinsing, cleaning and/or immersing therein a contact lens. Preferably, the sterile composition comprises an ophthalmically acceptable carrier, and may optionally be allowed to remain on the contact lens following rinsing, cleaning and/or immersing in the solution, as the residual solution will not harm the eye when the contact lens is placed on the eye. In some alternative embodiments, the aqueous carrier is not an ophthalmically acceptable carrier (e.g., wherein the carrier comprises a preservative and/or a concentration of preservative which is not ophthalmically acceptable), and the sterile composition (e.g., a composition having a sterile contact lens immersed therein according to any of the respective embodiments described herein) may optionally be for immersing a contact lens for an extended period of time (e.g., when the contact lens is not in use, for example, at night) and/or for storage for an extended period of time (e.g., between contact lens manufacture and initial use), while limiting the risk of bacterial growth in the solution; for example, wherein such a composition is rinsed with an ophthalmically acceptable liquid (e.g., water, saline) solution prior to placing the contact lens on the eye.

In an embodiment of the present invention described herein relating to a contact lens, the sterile composition is for immersing therein a contact lens (e.g., to maintain moisture of a contact lens, optionally while also reducing a friction coefficient of the contact lens). In some such embodiments, the carrier of the sterile composition comprises additional ingredients suitable for effecting cleaning, for example, a preservative. Such a composition may optionally be provided together with a contact lens immersed therein as a single product, e.g., wherein a sterile contact lens and sterile composition are packaged together. The sterile composition may optionally be intended to be rinsed off by a more ophthalmically acceptable composition prior to insertion into the eye.

In an embodiment of the present invention described herein relating to a contact lens, the sterile composition is for rinsing a contact lens (e.g., to remove another composition from the contact lens, such a liquid which is not ophthalmically acceptable, and/or to reduce a friction coefficient of the contact lens). Such a composition may optionally be provided as a separate product from the contact lens. In some such embodiments, the carrier of the sterile composition is an ophthalmically acceptable carrier.

In an embodiment of the present invention described herein relating to a contact lens, the sterile composition is for cleaning a used and/or new contact lens (e.g., to remove bacteria and/or other impurities, optionally while also reducing a friction coefficient of the contact lens). In some such embodiments, the carrier of the sterile composition comprises additional ingredients suitable for effecting cleaning, for example, an antimicrobial agent (e.g., a peroxide and/or other oxidizing agent) and/or a detergent for removing impurities is an ophthalmically acceptable carrier. Such a composition may optionally be provided as a separate product from the contact lens, which may optionally be intended to be rinsed off by a more ophthalmically acceptable composition prior to insertion into the eye.

In the present invention, the sterile composition according to any of the respective embodiments described herein is for use in the treatment of a synovial joint disorder, for example, wherein the treatment comprises intra-articular administration of the composition.

According to an aspect of the invention, there is provided a use of a sterile composition according to any of the respective embodiments described herein in the manufacture of a medicament for the treatment of a synovial joint disorder wherein the treatment comprises intra-articular administration of the composition.

Examples of synovial joint disorders treatable according to embodiments of various aspects of the invention, include, without limitation, arthritis (e.g., osteoarthritis, rheumatoid arthritis and/or psoriatic arthritis), bursitis, carpal tunnel syndrome, fibromyositis, gout, locked joint (optionally associated with osteochondritis dissecans and/or synovial osteochondromatosis), tendinitis, traumatic joint injury (optionally caused directly by trauma, e.g., inflicted at the time of the trauma, and/or by previous trauma, e.g., a post-traumatic injury which develops sometime after the trauma), and joint injury associated with surgery (optionally associated with surgery which directly inflicts damage on an articular surface, e.g., by incision, and/or surgery which damages an articular surface only indirectly; for example, surgery which repairs or otherwise affects tissue in the vicinity of the joint, such as ligaments and/or menisci, may be associated with joint injury due to altered mechanics in the joint). Osteoarthritis is an exemplary synovial joint disorder treatable according to some embodiments of the invention.

In some of embodiments, treatment of a synovial joint disorder (e.g., osteoarthritis) is characterized by a reduction in pain, for example, upon movement, at night, and/or at rest.

In such embodiments, reduction in pain may optionally be determined by any suitable technique known in the art. Examples of suitable techniques for determining reduction in pain include, without limitation, Brief Pain Inventory (e.g., short form), physical activity test (e.g., timed up and go), VAS (Visual Analogue Scale) questionnaire for assessing pain (no pain to unbearable pain), WOMAC (Western Ontario and McMaster Universities) scale, and/or KOOS (Knee Injury and Osteoarthritis Outcome Score).

In some embodiments, treatment of a synovial joint disorder (e.g., osteoarthritis) is characterized by an improvement in articular physiology.

In some embodiments, improvement in articular physiology is determined by a Kellgren-Lawrence scale of radiological severity, e.g., wherein the improvement is characterized as a reduction in severity. In some such embodiments, the treatment is further characterized by a reduction in pain (e.g., according to any of the respective embodiments described herein).

In some embodiments, improvement in articular physiology is determined by range of motion of an afflicted joint (e.g., wherein the improvement is characterized as an increase in range of motion of the joint), optionally in addition to being characterized as a reduction in severity according to a Kellgren-Lawrence scale. In some such embodiments, the treatment is further characterized by a reduction in pain (e.g., according to any of the respective embodiments described herein).

In some embodiments, improvement in articular physiology is characterized by an increase in physical activity (e.g., activity involving the afflicted joint), optionally in addition to being characterized as a reduction in severity according to a Kellgren-Lawrence scale and/or as an increase in range of motion (e.g., according to any of the respective embodiments described herein). In some such embodiments, the treatment is further characterized by a reduction in pain (e.g., according to any of the respective embodiments described herein).

In some embodiments, improvement in articular physiology is characterized by an increase in quality of life, optionally in addition to being characterized as a reduction in severity according to a Kellgren-Lawrence scale, an increase in range of motion and/or an increase in physical activity (e.g., according to any of the respective embodiments described herein). In some such embodiments, the treatment is further characterized by a reduction in pain (e.g., according to any of the respective embodiments described herein).

In some of embodiments, improvement in articular physiology is determined by at least one, or at least two, or at least three or all four of a Kellgren-Lawrence scale of radiological severity, range of motion of joint, physical activity, and quality of life (according to any of the respective embodiments described herein). In some such embodiments, the treatment is further characterized by a reduction in pain (e.g., according to any of the respective embodiments described herein).

In some of embodiments, the sterile composition according to any of the embodiments described herein is capable of improving knee histopathology in an animal model of osteoarthritis following intra-articular injection of the composition. Any suitable animal model of osteoarthritis known in the art may optionally be used. Examples of suitable animal models include, without limitation, a Hartley guinea pig model and a rat medial meniscal destabilization model (optionally performed as described in the Examples section herein). In some embodiments, the animal model is a rat animal model, for example, a rat medial meniscal destabilization model (optionally performed as described in the Examples section herein).

A composition for use in treating a synovial joint disorder (e.g., osteoarthritis) according to the present invention herein may optionally comprise one or more therapeutically active agent (e.g., according to any of the respective embodiments described herein). Examples of therapeutically active agents suitable for a composition for treating a synovial joint disorder include, without limitation, analgesics and anti-inflammatory agents.

Examples of suitable analgesics include, without limitation, allylprodine, alphamethylfentanyl, AP-237, bezitramide, butorphanol, buprenorphine, carfentanyl, clonidine, codeine, desmethylprodine, dextromoramide, dexocine, difenoxin, dihydrocodeine, dihydroetorphine, dihydromorphine, diphenoxylate, dipipanone, eluxadoline, ethylmorphine, etorphine, fentanyl, heterocodeine, hydrocone, hydromorphone, ketamine, ketobemidone, lefetamine, levomethadyl (e.g., levomethadyl acetate), levomethorphan, levorphanol, loperamide, meptazinol, methadone, mexiletine, mitragynine, morphine, nalbuphine, ohmefentanyl, oxycodone, oxymorphone, paracetamol, pentazocine, pethidine, phenethylphenylacetoxypiperidine, piritramide, prodine, promedol, propoxyphene, remifentanil, sulfentanil, tapentadol, tilidine, and tramadol.

Non-steroidal anti-inflammatory agents (e.g., a non-steroidal anti-inflammatory agent described herein), as well as steroidal anti-inflammatory agents, may also be used as an analgesic.

Examples of suitable anti-inflammatory agents include, without limitation, alclofenac; alclometasone (e.g., alclometasone dipropionate); algestone (e.g., algestone acetonide); alpha amylase; amcinafal; amcinafide; amfenac (e.g., amfenac sodium); amiprilose (e.g., amiprilose hydrochloride); anakinra; anirolac; anitrazafen; apazone; aspirin; balsalazide disodium; bendazac; benoxaprofen; benzydamine (e.g., benzydamine hydrochloride); bromelains; broperamole; budesonide; carprofen; cicloprofen; cintazone; cliprofen; clobetasol (e.g., clobetasol propionate, clobetasone butyrate); clopirac; cloticasone (cloticasome propionate); cormethasone (cormethasone acetate); cortodoxone; deflazacort; desonide; desoximetasone; dexamethasone (e.g., dexamethasone dipropionate); diclofenac (e.g., diclofenac potassium, diclofenac sodium); diflorasone (e.g., diflorasone diacetate); diflumidone (e.g., diflumidone sodium); diflunisal; difluprednate; diftalone; drocinonide; endrysone; enlimomab; enolicam (e.g., enolicam sodium); epirizole; etodolac; etofenamate; felbinac; fenamole; fenbufen; fenclofenac; fenclorac; fendosal; fenpipalone; fentiazac; flazalone; fluazacort; flufenamic acid; flumizole; flunisolide (e.g., flunisolide acetate); flunixin (e.g., flunixin meglumine); fluocortin (e.g., fluorcortin butyl); fluorometholone (e.g., fluorometholone acetate); fluquazone; flurbiprofen; fluretofen; fluticasone (e.g., fluticasone propionate); furaprofen; furobufen; halcinonide; halobetasol (e.g., halobetasol propionate); halopredone (e.g., halopredone acetate); ibufenac; ibuprofen (e.g., ibuprofen aluminum, ibuprofen piconol); ilonidap; indomethacin (e.g., indomethacin sodium); indoprofen; indoxole; intrazole; isoflupredone (e.g., isoflupredone acetate); isoxepac; isoxicam; ketoprofen; lofemizole (e.g., lofemizole hydrochloride); lomoxicam; loteprednol (e.g., loteprednol etabonate); meclofenamate (e.g., meclofenamate sodium, meclofenamic acid); meclorisone (e.g., meclorisone dibutyrate); mefenamic acid; mesalamine; meseclazone; methylprednisolone (e.g., methylprednisolone suleptanate); momiflumate; nabumetone; naproxen (e.g., naproxen sodium); naproxol; nimazone; olsalazine (e.g., olsalazine sodium); orgotein; orpanoxin; oxaprozin; oxyphenbutazone; paranyline (e.g., paranyline hydrochloride); pentosan polysulfate (e.g., pentosan polysulfate sodium); phenbutazone (e.g., phenbutazone sodium glycerate); pirfenidone; piroxicam (e.g., piroxicam cinnamate, piroxicam olamine); pirprofen; prednazate; prifelone; prodolic acid; proquazone; proxazole (e.g., proxazole citrate); rimexolone; romazarit; salcolex; salicylate (e.g., salicylic acid); salnacedin; salsalate; sanguinarium (e.g., sanguinarium chloride); seclazone; sermetacin; sudoxicam; sulindac; suprofen; talmetacin; talniflumate; talosalate; tebufelone; tenidap (e.g., tenidap sodium); tenoxicam; tesicam; tesimide; tetrydamine; tiopinac; tixocortol (e.g., tixocortol pivalate); tolmetin (e.g., tolmetin sodium); triclonide; triflumidate; zidometacin; and zomepirac (e.g., zomepirac sodium).

Additional therapeutically active agents suitable for a composition for treating a synovial joint disorder include, without limitation, cannabinoids (e.g., cannabidiol) and other cannabis-derived substances.

Techniques for formulation and administration of compounds may be found in "Remington's Pharmaceutical Sciences" Mack Publishing Co., Easton, PA, latest edition.

Sterile compositions (e.g., solutions) according to any one of the embodiments of the present invention may be manufactured by processes well known in the art, e.g., by means of conventional mixing or dissolving processes.

Sterile compositions (e.g., solutions) for use in accordance with the present invention thus may be formulated in conventional manner using one or more physiologically acceptable carriers, which facilitate processing of the liposomes (and optionally other components of the composition described herein) into preparations which can be used pharmaceutically. Proper formulation is dependent upon the route of administration chosen.

For injection, the sterile composition may be formulated using a suitable aqueous carrier, preferably a physiologically compatible buffer such as Hank's solution, Ringer's solution, histidine buffer, or physiological saline buffer with or without organic solvents such as propylene glycol, polyethylene glycol.

The sterile composition described herein may be formulated for parenteral administration, e.g., by bolus injection or continuous infusion. Formulations for injection may be presented in unit dosage form, e.g., in ampoules or in multi-dose containers with optionally, an added preservative. The composition may be formulated as a suspension, solutions or emulsion (e.g., in an aqueous carrier described herein), and may contain formulatory agents such as suspending, stabilizing and/or dispersing agents.

The sterile composition may be formulated as an aqueous solution *per se.* Additionally, the sterile composition may be in the form of a suspension and/or emulsions (e.g., water-in-oil, oil-in-water or water-in-oil-in-oil emulsion), for example, in order to increase the viscosity of the formulation. Aqueous injection suspensions may contain substances, which increase the viscosity of the suspension, such as sodium carboxymethyl cellulose, sorbitol or dextran. Optionally, the suspension may also contain suitable stabilizers or agents, which increase the solubility and/or stability of the liposomes described herein, for example, to allow for the preparation of highly concentrated solutions.

The sterile composition may be formulated wherein the liposomes are contained in an amount effective to achieve the intended purpose, for example, an amount effective to prevent, alleviate or ameliorate symptoms of a disorder in the subject being treated.

The dosage may vary depending upon the dosage form employed, the route of administration utilized, and the location of administration (e.g., the volume and/or surface of the region contacted with the liposomes).

The amount of a composition to be administered will, of course, be dependent on the subject being treated, the severity of the affliction, the manner of administration, the judgment of the prescribing physician, etc.

Sterile compositions (e.g., solutions) according to embodiments of the present invention may, if desired, be presented in a pack or dispenser device, such as an FDA (the U.S. Food and Drug Administration) approved kit, which may contain one or more unit dosage forms containing the active ingredient(s) (e.g., liposomes described herein). The pack may, for example, comprise metal or plastic foil, such as, but not limited to a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Sterile compositions comprising liposomes, as described herein in any one of the respective embodiments, formulated in a physiologically acceptable carrier may also be prepared, placed in an appropriate container, and labeled for treatment of an indicated condition or diagnosis, as is detailed herein.

### Additional definitions:

Herein, the term "hydrocarbon" describes an organic moiety that includes, as its basic skeleton, a chain of carbon atoms, substituted mainly by hydrogen atoms. The hydrocarbon can be saturated or non-saturated, be comprised of aliphatic, alicyclic or aromatic moieties, and can optionally be substituted by one or more substituents (other than hydrogen). A substituted hydrocarbon may have one or more substituents, whereby each substituent group can independently be, for example, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, oxo, cyano, nitro, azo, azide, sulfonamide, carboxy, thiocarbamate, urea, thiourea, carbamate, amide, and hydrazine. The hydrocarbon can be an end group or a linking group, as these terms are defined herein. The hydrocarbon moiety is optionally interrupted by one or more heteroatoms, including, without limitation, one or more oxygen, nitrogen and/or sulfur atoms. **In** some embodiments described herein relating to a hydrocarbon, the hydrocarbon is not interrupted by any heteroatoms.

Preferably, and unless otherwise indicated, the hydrocarbon moiety has 1 to 20 carbon atoms. Whenever a numerical range; e.g., "1 to 20", is stated herein, it implies that the group, in this case the alkyl group, may contain 1 carbon atom, 2 carbon atoms, 3 carbon atoms, etc., up to and including 20 carbon atoms.

Herein, the term "alkyl" refers to any saturated aliphatic hydrocarbon including straight chain and branched chain groups. Preferably, the alkyl group has 1 to 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or non-substituted. When substituted, the substituent group can be, for example, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

Herein, the term "alkenyl" describes an unsaturated aliphatic hydrocarbon comprise at least one carbon-carbon double bond, including straight chain and branched chain groups. Preferably, the alkenyl group has 2 to 20 carbon atoms. More preferably, the alkenyl is a medium size alkenyl having 2 to 10 carbon atoms. Most preferably, unless otherwise indicated, the alkenyl is a lower alkenyl having 2 to 4 carbon atoms. The alkenyl group may be substituted or non-substituted. Substituted alkenyl may have one or more substituents, whereby each substituent group can independently be, for example, alkynyl, cycloalkyl, alkynyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino.

Herein, the term "alkynyl" describes an unsaturated aliphatic hydrocarbon comprise at least one carbon-carbon triple bond, including straight chain and branched chain groups. Preferably, the alkynyl group has 2 to 20 carbon atoms. More preferably, the alkynyl is a medium size alkynyl having 2 to 10 carbon atoms. Most preferably, unless otherwise indicated, the alkynyl is a lower alkynyl having 2 to 4 carbon atoms. The alkynyl group may be substituted or non-substituted. Substituted alkynyl may have one or more substituents, whereby each substituent group can independently be, for example, cycloalkyl, alkenyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino.

The term "alkylene" describes a saturated or unsaturated aliphatic hydrocarbon linking group, as this term is defined herein, which differs from an alkyl group (when saturated) or an alkenyl or alkynyl group (when unsaturated), as defined herein, only in that alkylene is a linking group rather than an end group.

A "cycloalkyl" group refers to a saturated on unsaturated all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein. When a cycloalkyl group is unsaturated, it may comprise at least one carbon-carbon double bond and/or at least one carbon-carbon triple bond. The cycloalkyl group can be an end group, as this phrase is defined herein, wherein it is attached to a single adjacent atom, or a linking group, as this phrase is defined herein, connecting two or more moieties.

An "aryl" group refers to an all-carbon monocyclic or fused-ring polycyclic (i.e., rings which share adjacent pairs of carbon atoms) end groups having a completely conjugated pi-electron system. Examples, without limitation, of aryl groups are phenyl, naphthalenyl and anthracenyl. The aryl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

A "heteroaryl" group refers to a monocyclic or fused ring (i.e., rings which share an adjacent pair of atoms) end group having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazole, pyridine, pyrimidine, quinoline, isoquinoline and purine. The heteroaryl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

The term "arylene" describes a monocyclic or fused-ring polycyclic linking group, as this term is defined herein, and encompasses linking groups which differ from an aryl or heteroaryl group, as these groups are defined herein, only in that arylene is a linking group rather than an end group.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or non-substituted. When substituted, the substituted group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein. Representative examples are piperidine, piperazine, tetrahydrofuran, tetrahydropyran, morpholine and the like. The heteroalicyclic group can be an end group, as this phrase is defined herein, wherein it is attached to a single adjacent atom, or a linking group, as this phrase is defined herein, connecting two or more moieties.

Herein, the terms "amine" and "amino" each refer to either a -NR'R'' group or a - N⁺R'R"R‴ group, wherein R', R" and R‴ are each hydrogen or a substituted or non-substituted alkyl, alkenyl, alkynyl, cycloalkyl, heteroalicyclic (linked to amine nitrogen via a ring carbon thereof), aryl, or heteroaryl (linked to amine nitrogen via a ring carbon thereof), as defined herein. Optionally, R', R" and R‴ are hydrogen or alkyl comprising 1 to 4 carbon atoms. Optionally, R' and R" (and R‴, if present) are hydrogen. When substituted, the carbon atom of an R', R" or R‴ hydrocarbon moiety which is bound to the nitrogen atom of the amine is not substituted by oxo (unless explicitly indicated otherwise), such that R', R" and R‴ are not (for example) carbonyl, C-carboxy or amide, as these groups are defined herein.

An "azide" group refers to a -N=N⁺=N⁻ end group.

An "alkoxy" group refers to any of an -O-alkyl, -O-alkenyl, -O-alkynyl, -O-cycloalkyl, and -O-heteroalicyclic end group, as defined herein, or to any of an -O-alkylene, -O-cycloalkyl- and - O-heteroalicyclic- linking group, as defined herein.

An "aryloxy" group refers to both an -O-aryl and an -O-heteroaryl group, as defined herein, or to an -O-arylene.

A "hydroxy" group refers to a -OH group.

A "thiohydroxy" or "thiol" group refers to a -SH group.

A "thioalkoxy" group refers to any of an -S-alkyl, -S-alkenyl, -S-alkynyl, -S-cycloalkyl, and -S-heteroalicyclic end group, as defined herein, or to any of an -S-alkylene-, -S-cycloalkyl- and -S-heteroalicyclic- linking group, as defined herein.

A "thioaryloxy" group refers to both an -S-aryl and an -S-heteroaryl group, as defined herein, or to an -S-arylene.

A "carbonyl" or "acyl" group refers to a -C(=O)-R' end group, where R' is defined as hereinabove, or to a -C(=O)- linking group.

A "thiocarbonyl" group refers to a -C(=S)-R' end group, where R' is as defined herein, or to a -C(=S)- linking group.

A "carboxy", "carboxyl", "carboxylic" or "carboxylate" group refers to both "C-carboxy" and "O-carboxy" end groups, as defined herein, as well as to a carboxy linking group, as defined herein.

A "C-carboxy" group refers to a -C(=O)-O-R' group, where R' is as defined herein.

An "O-carboxy" group refers to an R'C(=O)-O- group, where R' is as defined herein.

A "carboxy linking group" refers to a -C(=O)-O- linking group.

An "oxo" group refers to a =O end group.

An "imine" group refers to a =N-R' end group, where R' is as defined herein, or to an =N-linking group.

An "oxime" group refers to a =N-OH end group.

A "hydrazone" group refers to a =N-NR'R" end group, where each of R' and R" is as defined herein, or to a =N-NR'- linking group where R' is as defined herein.

A "halo" group refers to fluorine, chlorine, bromine or iodine.

A "sulfinyl" group refers to an -S(=O)-R' end group, where R' is as defined herein, or to an -S(=O)- linking group.

A "sulfonyl" group refers to an -S(=O)₂-R' end group, where R' is as defined herein, or to an -S(=O)₂- linking group.

A "sulfonate" group refers to an -S(=O)₂-O-R' end group, where R' is as defined herein, or to an -S(=O)₂-O- linking group.

A "sulfate" group refers to an -O-S(=O)₂-O-R' end group, where R' is as defined as herein, or to an -O-S(=O)₂-O- linking group.

A "sulfonamide" or "sulfonamido" group encompasses both S-sulfonamido and N-sulfonamido end groups, as defined herein, as well as a sulfonamide linking group, as defined herein.

An "S-sulfonamido" group refers to a -S(=O)₂-NR'R" end group, with each of R' and R" as defined herein.

An "N-sulfonamido" group refers to an R'S(=O)₂-NR"- end group, where each of R' and R'' is as defined herein.

A "sulfonamide linking group" refers to a -S(=O)₂-NR'- linking group, where R' is as defined herein.

A "carbamyl" group encompasses both O-carbamyl and N-carbamyl end groups, as defined herein, as well as a carbamyl linking group, as defined herein.

An "O-carbamyl" group refers to an -OC(=O)-NR'R" end group, where each of R' and R" is as defined herein.

An "N-carbamyl" group refers to an R'OC(=O)-NR"- end group, where each of R' and R" is as defined herein.

A "carbamyl linking group" refers to a -OC(=O)-NR'- linking group, where R' is as defined herein.

A "thiocarbamyl" group encompasses O-thiocarbamyl, S-thiocarbamyl and N-thiocarbamyl end groups, as defined herein, as well as a thiocarbamyl linking group, as defined herein.

An "O-thiocarbamyl" group refers to an -OC(=S)-NR'R" end group, where each of R' and R" is as defined herein.

An "N-thiocarbamyl" group refers to an R'OC(=S)NR"- end group, where each of R' and R" is as defined herein.

An "S-thiocarbamyl" group refers to an -SC(=O)-NR'R" end group, where each of R' and R" is as defined herein.

A "thiocarbamyl linking group" refers to a -OC(=S)-NR'- or -SC(=O)-NR'- linking group, where R' is as defined herein.

An "amide" or "amido" group encompasses C-amido and N-amido end groups, as defined herein, as well as an amide linking group, as defined herein.

A "C-amido" group refers to a -C(=O)-NR'R" end group, where each of R' and R" is as defined herein.

An "N-amido" group refers to an R'C(=O)-NR"- end group, where each of R' and R" is as defined herein.

An "amide linking group" refers to a -C(=O)-NR'- linking group, where R' is as defined herein.

A "urea group" refers to an -N(R')-C(=O)-NR"R‴ end group, where each of R', R" and R" is as defined herein, or an -N(R')-C(=O)-NR"- linking group, where each of R' and R" is as defined herein.

A "thiourea group" refers to an -N(R')-C(=S)-NR"R‴ end group, where each of R', R" and R" is as defined herein, or an -N(R')-C(=S)-NR"- linking group, where each of R' and R" is as defined herein.

A "nitro" group refers to an -NO₂ group.

A "cyano" group refers to a -C≡N group.

The term "phosphonyl" or "phosphonate" describes a -P(=O)(OR')(OR") group, with R' and R" as defined herein, or a -P(=O)(OR')-O- linking group, with R' as defined herein.

The term "phosphate" describes an -O-P(=O)(OR')(OR") end group, with each of R' and R" as defined herein, or an -O-P(=O)(OR')-O- linking group, with R' as defined herein.

The term "phosphinyl" describes a -PR'R" end group, with each of R' and R" as defined herein, or a -PR'- linking group, with R' as defined herein.

The term "hydrazine" describes a -NR'-NR"R‴ end group, where R', R", and R‴ are as defined herein, or to a -NR'-NR"- linking group, where R' and R" are as defined herein.

As used herein, the term "hydrazide" describes a -C(=O)-NR'-NR"R‴ end group, where R', R" and R‴ are as defined herein, or to a -C(=O)-NR'-NR"- linking group, where R' and R" are as defined herein.

As used herein, the term "thiohydrazide" describes a -C(=S)-NR'-NR"R‴ end group, where R', R" and R‴ are as defined herein, or to a -C(=S)-NR'-NR"- linking group, where R' and R" are as defined herein.

A "guanidinyl" group refers to an -RaNC(=NRd)-NRbRc end group, where each of Ra, Rb, Rc and Rd can be as defined herein for R' and R", or to an -R'NC(=NR")-NR'''- linking group, where R', R" and R‴ are as defined herein.

A "guanyl" or "guanine" group refers to an R‴R"NC(=NR')- end group, where R', R" and R‴ are as defined herein, or to a -R"NC(=NR')- linking group, where R' and R" are as defined herein.

According to the present invention, the compound described herein may be in a form of a salt, for example, a pharmaceutically acceptable salt.

As used herein, the phrase "pharmaceutically acceptable salt" refers to a charged species of the parent compound and its counter-ion, which is typically used to modify the solubility characteristics of the parent compound and/or to reduce any significant irritation to an organism by the parent compound, while not abrogating the biological activity and properties of the administered compound. A pharmaceutically acceptable salt of a compound as described herein can alternatively be formed during the synthesis of the compound, e.g., in the course of isolating the compound from a reaction mixture or re-crystallizing the compound.

In the context of the present embodiments, a pharmaceutically acceptable salt of the compounds described herein may optionally be an acid addition salt and/or a base addition salt.

An acid addition salt comprises at least one basic (e.g., amine and/or guanidinyl) group of the compound which is in a positively charged form (e.g., wherein the basic group is protonated), in combination with at least one counter-ion, derived from the selected acid, that forms a pharmaceutically acceptable salt. The acid addition salts of the compounds described herein may therefore be complexes formed between one or more basic groups of the compound and one or more equivalents of an acid.

A base addition salt comprises at least one acidic (e.g., carboxylic acid) group of the compound which is in a negatively charged form (e.g., wherein the acidic group is deprotonated), in combination with at least one counter-ion, derived from the selected base, that forms a pharmaceutically acceptable salt. The base addition salts of the compounds described herein may therefore be complexes formed between one or more acidic groups of the compound and one or more equivalents of a base.

Depending on the stoichiometric proportions between the charged group(s) in the compound and the counter-ion in the salt, the acid additions salts and/or base addition salts can be either mono-addition salts or poly-addition salts.

The phrase "mono-addition salt", as used herein, refers to a salt in which the stoichiometric ratio between the counter-ion and charged form of the compound is 1:1, such that the addition salt includes one molar equivalent of the counter-ion per one molar equivalent of the compound.

The phrase "poly-addition salt", as used herein, refers to a salt in which the stoichiometric ratio between the counter-ion and the charged form of the compound is greater than 1:1 and is, for example, 2:1, 3:1, 4:1 and so on, such that the addition salt includes two or more molar equivalents of the counter-ion per one molar equivalent of the compound.

An example, without limitation, of a pharmaceutically acceptable salt would be an ammonium cation or guanidinium cation and an acid addition salt thereof, and/or a carboxylate anion and a base addition salt thereof.

The base addition salts may include a cation counter-ion such as sodium, potassium, ammonium, calcium, magnesium and the like, that forms a pharmaceutically acceptable salt.

The acid addition salts may include a variety of organic and inorganic acids, such as, but not limited to, hydrochloric acid which affords a hydrochloric acid addition salt, hydrobromic acid which affords a hydrobromic acid addition salt, acetic acid which affords an acetic acid addition salt, ascorbic acid which affords an ascorbic acid addition salt, benzenesulfonic acid which affords a besylate addition salt, camphorsulfonic acid which affords a camphorsulfonic acid addition salt, citric acid which affords a citric acid addition salt, maleic acid which affords a maleic acid addition salt, malic acid which affords a malic acid addition salt, methanesulfonic acid which affords a methanesulfonic acid (mesylate) addition salt, naphthalenesulfonic acid which affords a naphthalenesulfonic acid addition salt, oxalic acid which affords an oxalic acid addition salt, phosphoric acid which affords a phosphoric acid addition salt, toluenesulfonic acid which affords a p-toluenesulfonic acid addition salt, succinic acid which affords a succinic acid addition salt, sulfuric acid which affords a sulfuric acid addition salt, tartaric acid which affords a tartaric acid addition salt and trifluoroacetic acid which affords a trifluoroacetic acid addition salt. Each of these acid addition salts can be either a mono-addition salt or a poly-addition salt, as these terms are defined herein.

Further, each of the compounds described herein, including the salts thereof, can be in a form of a solvate or a hydrate thereof.

The term "solvate" refers to a complex of variable stoichiometry (e.g., di-, tri-, tetra-, penta-, hexa-, and so on), which is formed by a solute (the heterocyclic compounds described herein) and a solvent, whereby the solvent does not interfere with the intended activity of the solute.

The term "hydrate" refers to a solvate, as defined hereinabove, where the solvent is water.

The compounds described herein can be used as polymorphs and the present embodiments further encompass any isomorph of the compounds and any combination thereof.

The compounds and structures described herein encompass any stereoisomer, including enantiomers and diastereomers, of the compounds described herein, unless a particular stereoisomer is specifically indicated.

As used herein, the term "enantiomer" refers to a stereoisomer of a compound that is superposable with respect to its counterpart only by a complete inversion/reflection (mirror image) of each other. Enantiomers are said to have "handedness" since they refer to each other like the right and left hand. Enantiomers have identical chemical and physical properties except when present in an environment which by itself has handedness, such as all living systems. In the context of the present embodiments, a compound may exhibit one or more chiral centers, each of which exhibiting an (R) or an (S) configuration and any combination, and compounds according to some embodiments of the present invention, can have any their chiral centers exhibit an (R) or an (S) configuration.

The term "diastereomers", as used herein, refers to stereoisomers that are not enantiomers to one another. Diastereomerism occurs when two or more stereoisomers of a compound have different configurations at one or more, but not all of the equivalent (related) stereocenters and are not mirror images of each other. When two diastereoisomers differ from each other at only one stereocenter they are epimers. Each stereo-center (chiral center) gives rise to two different configurations and thus to two different stereoisomers. In the context of the present invention, embodiments of the present invention encompass compounds with multiple chiral centers that occur in any combination of stereo-configuration, namely any diastereomer.

As used herein the term "about" refers to ± 10 %, and optionally ± 5 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

### EXAMPLES

### MATERIALS AND METHODS

### Synthesis and characterization of DPPE-Br (1,2-dipalmitoyl-sn-glycero-3-phosphoethanolamine-2'-bromoisobutyrate):

DPPE-Br (1,2-dipalmitoyl-sn-glycero-3-phosphoethanolamine-2'-bromoisobutyrate) was prepared according to procedures described in Li et al. [J Controlled Release 2014, 176:104-114]. DPPE lipid was mixed with dichloromethane (DCM) in a flask, shaken by hand to obtain a white suspension. Triethylamine was added to the flask, and the mixture was stirred until complete mixing was achieved. α-Bromoisobutyryl bromide was then added carefully to the suspension. The solution was then washed with HCl using a separatory funnel. The organic phase was dehydrated with Na₂SO₄ and the volume of the target solution was reduced using a rotary evaporator. The final product was isolated by adding the DCM solution to cold methanol. Precipitation lasted overnight in the freezer. A white powder of DPPE-Br was filtered and dried in a desiccator for 3 days. The final product was characterized by ¹H NMR (300 MHz, CDCl₃): δ=5.25(-OCHCH₂O-P-); δ=4.45 (-CH₂COOCH₂-); δ=4.30 (-P-OCH₂CH₂-); δ=4.14 (-P-OCH₂CH-); δ=3.57 (-OCH₂CH₂N-); δ=2.30 (-COCH₂CH₂-); δ=1.95 (-BrCCH₃CH₃); δ=1.60 (-COCH₂CH₂-); δ=1.25 (-(CH₂)₁₄-); δ=0.88 (-CH₂CH₃).

### Synthesis and characterization of DPPE-pMPC (1,2-dipalmitoyl-sn-glycero-3-phosphoethanolamine-poly(2-(methacryloyloxy)ethyl phosphorylcholine):

The general scheme for the synthesis of lipid-conjugated polymer DPPE-pMPC is as described in Lin et al. [Langmuir 2019, 35:6048-6054]. DPPE-Br initiator was mixed with MPC monomer in ethanol, and then PMDETA (N,N,N',N",N"-pentamethyldiethylenetriamine) ligand was added to the flask. Atom transfer radical polymerization was triggered by the addition of the catalyst CuBr. At the end of polymerization, reaction mixture was precipitated in cold diethyl ether, a blue solid intermediate was filtered and re-dissolved in ethanol. Ethanol solution was added drop by drop to water with stirring, and then HCl was added to obtain a clear colorless solution. TFF (tangential flow filtration) cassette cleaning was performed to cut off the low molecular weight compounds. The final product was obtained after freeze-drying. The lipid-conjugated polymer was characterized by ¹H NMR (300 MHz, CDCl₃: CD₃OD 1:1): δ=5.25 (-OCHCH₂O-P-); δ=4.32 (-N-CH₂CH₂O-); δ=4.22 (-CO-OCH₂CH₂-); δ=4.07 (-OCH₂CH₂O-P-); δ=3.75 (-(CH₃)₃N⁺-CH₂CH₂-); δ=3.34 (-(CH₃)₃N⁺-); δ=1.9 (-(CH₂)ₙ-, active center); δ=1.28 (-(CH₂)₁₂-); δ=0.88 (-CH₂CH₃). Molecular weight was determined by the integration of peaks with chemical shifts δ=1.28 and δ=3.75, corresponding to the relative amount of hydrogen atoms in the lipid and monomer parts.

Further data regarding preparation and characterization of a lipid-conjugated polymer such as DPPE-pMPC can be found in WO 2017/109784.

### EXAMPLE 1

### Preparation of sterile liposome composition

Liposomes were prepared following the hydration-extrusion technique, according to procedures such as described in Cao et al. [Langmuir 2012, 28:11625-11632]. Phosphatidylcholine and DPPE-pMPC were dissolved in heated organic solvent. Solvent was removed using a rotary evaporator to obtain a dry homogeneous lipid mixture. The obtained lipid powder was dissolved in ethanol. Multilamellar vesicles (MLVs) are formed by filtration of an ethanol solution with a 0.2 µm filter directly into hot aqueous medium. The liposome suspension was stirred at 65 °C for at least 25 minutes. MLVs were passed through membrane filters with a defined pore size of 400 nm and 200 nm in order to downsize the vesicle size. Extrusion was repeated to obtain small unilamellar vesicles (SUVs).

A TFF system was used to remove the low molecular weight fraction.

The liposome solution was filled into pre-sterilized glass vials of 5 ml, each vial was closed with a rubber stopper and sealed with aluminum caps. The liposome product in the primary packaging was steam sterilized at 121 °C for 20 minutes. The efficacy of this sterilization procedure was confirmed by the absence of microbial growth according to the sterility test results; as well as the presence of less than 25 EU/ml of endotoxins (acceptance criteria for bacterial endotoxin test is <35 EU/ml).

The osmolality of the liposome solution was determined by micro-osmometer (Standalone Instrument) to be 289 mOsm/kg, which is consistent with small unilamellar vesicles being not osmotically active.

The size distribution and zeta potential of liposomes in the sterilized solution were determined by dynamic light scattering, using a Zetasizer^{™} Nano ZS device (Malvern). Zeta potential was measured upon dilution of the samples in a 10 µM salt solution.

Size distribution and zeta potential data are presented in FIG. 1 and Table 1 below.

**Table 1: Initial size distribution and zeta potential of a 1 mM liposome solution**

| | **Mean** | **Standard deviation** | **Relative standard deviation** | **Minimum** | **Maximum** |
|---|---|---|---|---|---|
| Diameter (Z-average) | 165 nm | 0.638 nm | 0.386 % | 165 nm | 166 nm |
| Polydispersity index (diameter) | 0.0606 | 0.0135 | 22.2 % | 0.047 | 0.0739 |
| Diameter (peak) | 178 nm | 3.05 nm | 1.71 % | 175 nm | 181 nm |
| Zeta potential | -3.95 mV | 1.05 mV | 26.5 % | -5.16 mV | -3.29 mV |

In order to visualize the liposomes and characterize their size and shape, cryo-tunneling electron microscopy was performed by fast freezing an exemplary liposome composition on a carbon grid.

As shown in FIGs. 2A and 2B, the liposomes were generally spherical in shape, and included unilamellar and multilamellar structures, with a heterogeneous size distribution of 50 to 200 nm in diameter, as determined by cryo-tunneling electron microscopy.

As shown in FIGs. 3 and 4, the Z-average of liposome diameter (FIG. 3) and polydispersity index (FIG. 4) of the sterilized liposome solution was stable over the course of 350 days (storage at room temperature), as determined by dynamic light scattering.

These results indicate that the sterilized liposome solution is highly stable even after steam sterilization, despite the hydrolytic degradation of liposomes which may be induced by steam sterilization [Toh & Chiu, Asian J Pharm Sci 2013, 8:88-95]. This is advantageous, as steam sterilization is typically cheaper and more convenient and safe than alternative sterilization procedures such as γ-irradiation, ethylene oxide, UV sterilization etc.

### EXAMPLE 2

### Effect of exemplary sterile liposome composition on lubricity in simulated knee model

The effect of an exemplary sterile composition, prepared as described in Example 1 on the lubricity of a simulated knee, using a pin-on-disk test with duration, loading and waveform parameters in accordance with ASTM F732 standards, over the course of 500,000 cycles. Friction coefficients were determined over the course of the 500,000 cycles, and wear rate was evaluated by determining the weight loss of the pin after 500,000 cycles. The effect of the exemplary composition as a lubricant was compared with a bovine calf serum (BCS)-based lubricant (mimicking human synovial fluid) and 50:50 and 25:75 mixtures of the exemplary composition and BCS.

As shown in Table 2 below and in FIG. 5, the exemplary composition (*per se* and in admixture with BCS) provided lower average and maximum friction coefficients than did BCS.

As further shown in Table 2, the exemplary composition considerably reduced the degree of wear (weight loss of the pin) after 500,000 cycles, as compared with BCS, in a dose-dependent manner.

These results indicate that the sterile compositions described herein are suitable for enhancing lubricity of joints.

**Table 2: Friction coefficient and wear (weight loss) of simulated knee in the presence of exemplary liposome-containing composition, bovine calf serum or mixtures thereof**

| | **Liposome-containing composition (100 %)** | **Liposome-containing composition (50 %) + bovine calf serum (50 %)** | | **Liposome-containing composition (25 %) + bovine calf serum (75 %)** | **Bovine calf serum (100 %)** | |
|---|---|---|---|---|---|---|
| Sample # | 1 | 2 | 3 | 4 | 5 | 6 |
| Maximum friction coefficient | 0.120 | 0.135 | 0.120 | 0.108 | 0.148 | 0.154 |
| Average (± standard deviation) friction coefficient (for sample) | 0.067 ± 0.018 | 0.074 ± 0.021 | 0.063 ± 0.022 | 0.061 ± 0.025 | 0.076 ± 0.033 | 0.076 ± 0.035 |
| Average friction coefficient (for composition type) | 0.067 | 0.069 | | 0.061 | 0.076 | |
| Wear (mg) | 0.377 | 0.910 | 0.957 | 3.550 | 6.163 | 2.913 |

### EXAMPLE 3

### Effect of exemplary sterile liposome composition on cartilage lubricity

The effect of an exemplary composition, prepared as described in Example 1 on the lubricity of a cartilage surface is evaluated *in vitro.* Fresh articular cartilage from knees of 1-year-old calves, are cut into a disk structure. The mechanical properties of the samples are determined by means of indentation assays, and the maximum experimental contact pressure is calculated, optionally for a pin-on-disk setup. Tribological measurements are performed in a speed that is representative for the physiological articular motion, e.g., 1 mm/second, for an hour. Friction coefficient is calculated by dividing the lateral measured force by the applied normal force during the shear. A control measurement is performed using, e.g., a PBS solution. The results are compared with the results of corresponding measurements performed using e.g. a high molecule weight hyaluronic acid or a commercial hyaluronic acid viscosupplement, such as a Synvisc^{®} composition.

### EXAMPLE 4

### Efficacy of exemplary sterile liposome composition in treating osteoarthritis in animal model

Light microscopic studies have shown that Hartley guinea pigs develop moderate to severe destruction of the cartilage and subchondral bone sclerosis between 6 and 12 months of age, predominantly in the central portion of the medial tibial plateau and that the changes regularly progress to severe osteoarthritis (OA) in old age [de Bri et al., J Orthop Res 1995, 13:769-776; de Bri et al., Acta Orthop Scan 1996, 67:498-504], with typical changes that mimic human disease [Bendele & Hulman, Arthritis Rheum 1988, 31:561-565]. Dunkin Hartley guinea pigs were therefore selected as a model of spontaneous, age-related osteoarthritis to perform an *in vivo* preclinical study of the effect of an exemplary composition prepared as described in Example 1 on articular lubrication in the knee upon intra-articular administration.

After one week of animal acclimatization, the test composition is administered by intra-articular injection. Animals are then monitored for 3 to 6 months, in order to assess the effect of the test composition. Histological analysis is primarily addressed to tissue lesions and eventual tissue regeneration. All stained slides are scanned using a NanoZoomer^{™} digital slide scanner (Hamamatsu) and converted into digital format for further analysis and OA scoring.

In another study, a rat medial meniscal destabilization model of osteoarthritis is used. Rats are randomized into groups, anesthetized with isoflurane and a surgically induced osteoarthritis procedure is performed on the right knee. The effect of intra-articularly administered test compositions on knee histopathology (e.g., chondrocyte death, cartilage degeneration) and pain 56 days post-surgery is evaluated.

The presence of reduction in one or more sign of disease described hereinabove is determined.

### EXAMPLE 5

### Efficacy of exemplary sterile liposome composition in treating osteoarthritis in humans

A Phase I open label study is performed on osteoarthritic volunteers (from 18 to 85 years in age (optionally from 40 to 80), body mass index from 18.5 to 35) with pain in the intended study knee characterized by an average VAS (Visual Analogue Scale) score (active) of more than 5 over the last week prior to screening, and with degenerative changes in the intended study knee that can be categorized as grade III-IV (Kellgren-Lawrence) based upon standing posterior-anterior and lateral radiographs of the knee [Kellgren & Lawrence, Ann Rheum Dis 1957, 16:494-502].

Subjects are subjected to a washout period for analgesics and NSAIDs lasting 2-5 days, depending on the drug, and then administered 4 ml of an exemplary composition prepared as described in Example 1 (also referred to herein as "AqueousJoint") by a single intra-articular injection into the target knee (the injection volume is based on the amount of standard sodium hyaluronate products that are commonly injected several times at weekly intervals), along with standard conservative treatment (e.g., 500 mg paracetamol for mild pain and 1 gram paracetamol for more severe pain). Patients are placed in a supine position with the eyes blinded; the knee is flexed approximately 60° and prepared in a sterile fashion; and a 21-gauge needle (0.8 × 50 mm) is inserted into the joint capsule. Follow up occurs at 4, 8, 12 and 24 weeks after administration.

Safety of the intra-articular injection in subjects for up to 6 months is evaluated by investigating adverse reactions related to the injected material, injection-related side effects such as injection-site reaction, erythema, swelling, injection-site pain, and pruritus. In addition, the effect of treatment on range of motion, functionality, life quality, and analgesics consumption may optionally be evaluated.

Diagnostic techniques for evaluating subject status include MRI analysis of treated knee (e.g., 4 and/or 24 weeks after treatment); grading by diagnosis; physical evaluation, e.g., for varus (bow-legs)/valgus (knock-knees), etiology of knee osteoarthritis (e.g., malaligned knee or anterior cruciate ligament injury), and/or primary compartment of knee affected (patellofemoral versus medial, tibiofemoral versus lateral tibiofemoral); radiographic severity by Kellgren-Lawrence scale; range of motion (measurement of knee flexion and extension using a universal goniometer with prone subject); pain evaluation, e.g., as determined by Brief Pain Inventory (short form); and/or physical activity test (e.g., timed up and go); recording of analgesic and/or antiinflammatory medications; recording adverse events and serious adverse events (i.e., requires hospitalization or prolongation of inpatient's hospitalization, in persistent or significant disability or incapacity, or is life-threating or results in death); and/or subject completing questionnaires such as VAS for assessing pain (no pain to unbearable pain), e.g., upon movement, at night, at rest; WOMAC (Western Ontario and McMaster Universities) scale for assessing pain, stiffness and physical activity; KOOS (Knee Injury and Osteoarthritis Outcome Score) for assessing pain, other symptoms, function in daily living, function in sport and recreation and knee related quality of life; and/or SF12 (for assessing health related quality of life) questionnaires.

The presence of reduction in one or more sign of disease described hereinabove is determined.

The study synopsis is outlined in Table 3 hereinbelow.

**Table 3: Human safety clinical trial - study synopsis:**

| **Study Title** | **AqueousJoint Intra-Articular Injection in Moderate Knee Osteoarthritis: Safety Study** | | |
|---|---|---|---|
| **Study Device** | AqueousJoint- intra-articular injectable joint lubricant | | |
| **Protocol number** | CJO-01 Revision 1.0 | | |
| **Investigational Device** | ***AqueousJoint*** | | |
| | AqueousJoint is a new intra-articular (IA) injectable joint lubricant for patients suffering from knee Osteoarthritis developed by Liposphere LTD. AqueousJoint is a liposomal boundary lubricant, which coats the cartilage surface and protects it from further damage and degradation. The product goal is restoring cartilage its natural biolubrication properties. | | |

| **Study Title** | **AqueousJoint Intra-Articular Injection in Moderate Knee Osteoarthritis: Safety Study** | | |
|---|---|---|---|
| | AqueousJoint is a surface lubricant that does not change the synovial fluid characterization, but provides hydration protection from cartilage degradation and wear. | | |
| | The device is produced under GMP standards and has full traceability. | | |
| **Indication for Use:** | Intended to serve as a boundary lubricant which coats the cartilage surface and protects it from further damage and degradation. | | |
| **Investigational treatment** | Subjects receive a single intra-articular injection of AqueousJoint. | | |
| **Study design** | Design: Single center, Open Label Study. | | |
| | Masking: NONE | | |
| | Prior to screening, the candidate signs and date the ICF approved by the IRB/EC in accordance with the regulations. | | |
| | | | |
| | Patients eligible at screening enter a washout period for analgesics and NSAIDs lasting 2 to 5 days, depending on the drug. They receive a single intra-articular injection of AqueousJoint (test product). | | |
| | | | |
| | Group: only one Group receiving Standard conservative treatment + single intra-articular injection of AqueousJoint. | | |
| | | | |
| | Treatment administration: | | |
| | Treatment product (4 ml) is administered via a single intra-articular injection into the target knee. The choice of the injection volume is based on the amount of standard sodium hyaluronate products that are commonly injected several times at weekly intervals. | | |
| | All procedures are performed in an outpatient clinic. Injections are performed by the senior author, who has experience of >100 cases per year in knee joint injections or aspirations. Patients are placed in a supine position with the eyes blinded. The knee is flexed approximately 60° and prepared in a sterile fashion. A 21-gauge needle (0.8 × 50 mm) is inserted into the joint capsule. | | |
| | | | |
| | Visits: | | |
| | Follow up takes place at 4, 8, 12 and 24 weeks. | | |
| **Study Duration** | Study duration is 12 months. | | |
| **Study population** | A total of up to 20 patients, Male and female, age ≥18 and ≤ 85 years old | | |
| **Objectives** | Primary objective | | |
| | | • To evaluate the safety of intra-articular injection of Aqueous Joint in osteoarthritic patients up to 6 months of follow-up in an open label clinical study. | |
| | | | The safety parameters will include: adverse reactions related to the injected material; injection-related side effects consisted of injection-site reaction, erythema, swelling, injection-site pain, and pruritus. |
| | Secondary objectives | | |
| | | | To measure the effect of the treatment on range of motion, functionality, life quality, and analgesics consumption |
| **Study End-Points** | | • Safety throughout the study as determined by the investigator by examining the post-treatment occurrences of complications and adverse events. The safety parameters will include: adverse reactions related to the injected material; injection-related side effects consisted of injection-site reaction, erythema, swelling, injection-site pain, and pruritus. | |
| **Inclusion Criteria:** | | 1. Subject has signed and dated the informed consent form | |
| | | 2. Age ≥18 and ≤ 85 years old | |
| | | 3. Pain in the intended study knee with an average VAS score (active) of >5 over the last week prior to screening. | |
| | | 4. Degenerative changes in the intended study knee that can be categorized as grade III-IV Kellgren Lawrence based upon standing posterior-anterior and lateral radiographs of the knee. | |
| | | 5. Body Mass Index (BMI) between 18.5 and 35 | |
| **Exclusion Criteria:** | | 1. History of significant knee trauma or previous arthroscopic surgery of the intended study knee within the last 3 months preceding screening | |
| | | 2. Pain in both knees with a VAS score of ≥5 | |
| | | 3. Intra-articular injection to the intended study knee within 3 months prior to Screening | |
| | | 4. Life expectancy of less than 12 month | |
| | | 5. Intake of chronic pain medications (especially opioid pain relievers) without an option to pause for the period of the study | |
| | | 6. History of Psoriatic Arthritis, Rheumatoid Arthritis or any other inflammatory condition associated with arthritis | |
| | | 7. Wound in the area of the intended study knee | |
| | | 8. Fever signs or symptoms of systemic infection or infection of the intended study knee, on the day before or the day of administration of treatment or placebo | |
| | | 9. Known sensitivity to any of the treatment components, egg, rubber or latex | |
| | | 10. Known Human Immunodeficiency Virus/Acquired Immunodeficiency Syndrome (HIV/AIDS), Hepatitis B or C viral | |
| | | 11. infections, or acute or chronic liver disease | |
| | | 12. History of cellulitis of the lower extremities, a peripheral vascular disease, or acute or chronic liver disease | |
| | | 13. Cancer in the past 3 years or surgery involving the chest, abdomen, pelvis, or lower extremities in the past year | |
| | | 14. Participate in other clinical trial within 3 months prior to the study or concurrent with the study | |

### EXAMPLE 6

### Efficacy of exemplary sterile liposome composition for treating osteoarthritis in a rat animal model

### Methods:

Rats were randomized into groups with 15 animals per group. On study day 0 the animals were anesthetized with isoflurane and a surgical procedure to destabilize the medial meniscus (DMM) was performed on the right knee of the animals. 50 µl of the exemplary composition prepared as described in Example 1 (also referred to herein as "AqueousJoint"), Synvisc^{®} (a commercial HA injectable product) or saline (as a vehicle) were locally injected intra-articularly (IA) at day 7, 21, 35 and 49 post-surgery according to the group. The animals were euthanized 56 days post-surgery. Over 56 days post-surgery, dynamic weight bearing, comparing the difference between the force applied on the operated leg to the non-treated leg was analyzed among all rats. For the purposes of immediate pain management, a dose of meloxicam (1 mg / kg) was given orally, between 30-60 minutes prior to the surgery as well as a follow-on dose 24 hours post-surgery. Clinical parameters, dynamic weight bearing [DWB, to evaluate nociceptive pain (inflammatory hyperalgesia/mechanical allodynia)] and gait (mobility) were assessed throughout the duration of the study. Gait was scored as follows: 0 = normal, 1 = slight, 2 = mild, 3 = moderate, 4 = marked, 5 = severe, 6 = hopping. Histopathologic evaluation of knees was performed to assess joint damage. All animals survived to study termination.

### Results:

A rat medial meniscal destabilization model of osteoarthritis was used as an *in vivo* preclinical model to study the effect of an exemplary composition prepared as described in Example 1 on treating osteoarthritis by restoring the natural lubrication properties of cartilage to enable smooth articulation within the affected joint. Without being bound by theory, the formulation acts as a long-lasting liposomal, highly hydrated boundary lubricant on cartilage surfaces; and the unique lipid-poly-phosphocholine conjugate incorporated into the liposomes to stabilizes against aggregation and acts as an extremely efficient lubricating element to allow for protection against wear and tear.

Rats across all groups showed a clear difference in weight bearing between the operated leg and the non-operated leg following the surgical induction procedure, as seen on day 7 in FIG. 6. While administration of Synvisc^{®} had a beneficial effect on day 28 following surgery, treatment with AqueousJoint was significantly better in terms of efficacy, showing almost equal weight bearing on both legs (FIG. 6 and Table 4 hereinbelow). 56 days following surgery, AqueousJoint performance remained stable, while the effect of Synvisc^{®} reverted to the weight bearing characteristics of the vehicle group (FIG. 6). As shown in FIG. 7 and Table 4 hereinbelow, the mobility of rats treated with AqueousJoint, as determined by the gait score at 10 days post-surgery, was significantly better compared to rats treated with Synvisc^{®} or vehicle control. In addition, treatment with Synvisc^{®} significantly increased synovitis scores as compared to vehicletreated controls (Table 4 hereinbelow).
Taken together, treatment with AqueousJoint in an osteoarthritis rat model was superior compared to the current market standard treatment.

**Table 4: Summary of Clinical and Histopathology Data**

| **Group** | **Treatment** | **Gait Analysis Score - Day 10** | **Dynamic Weight Bearing Difference AUC** | **Substantial Medial Tibial Cartilage Degeneration Width (µm)** | **Total Joint Score without Femur** | **Synovitis Score** |
|---|---|---|---|---|---|---|
| 1 | Vehicle (Saline) IA, d7, 21, 35, 49 | 0.77 (0.11) | 597.8 (165.3) | 513.33 (63.33) | 6.43 (0.61) | 0.43 (0.08) |
| 2 | Synvisc^{®} IA, d7, 21, 35, 49 | 0.62 (0.13) | 440.4 (154.9) | 473.33 (50.21) | 6.30 (0.56) | 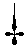 1.13 (0.15) |
| 3 | AqueousJoint IA, d7, 21, 35, 49 | 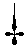 0.32 (0.08) | 224.6 (200.8) | 470.00 (62.64) | 5.93 (0.72) | 0.53 (0.06) |
| IN | Normal Left | -- | -- | *0.00 (0.00) | *0.10 (0.10) | *0.00 (0.00) |

| | | | | | | |
|---|---|---|---|---|---|---|
| Values represent Mean + Standard Error (SE), AUC = Area Under the Curve *p<0.05 Student's t-test or Mann-Whitney U test vs. Vehicle 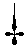 p<0.05 ANOVA/Lruskal-Wallis test (w/Dunnett's or Dunn's post-hoc) vs. Vehicle | | | | | | |

### EXAMPLE 7

### Tribological measurements

### Methods:

Measurements were conducted by means of a micro-tribometer. The evaluation was performed according to the standard procedure based on the work published by Roba et al. (Tribology Letters 2011, 44, 387-397). Briefly, under the assumption of a linear dependency between Fn and Ft, lateral and experimentally determined normal force values were calculated by averaging 20 data points at around 1.5 mm distance, and then plotted in a graph. Trace and retrace curves obtained in this way were averaged and the coefficient of friction was determined from the slope of linear regression of the selected points. Contact pressure was calculated using a Hertzian model and was calculated to be 5.6 MPa.

The comparative measurements were performed at a speed of 1 mm / sec, following a fivestep procedure: (i) PBS-Baseline, (ii) HA solution, (iii) PBS-rinsing, (iv) solution of the exemplary composition prepared as described in Example 1 (also referred to herein as "Synthetic Lipid solution"), (v) PBS-rinsing. After each step, the system was rinsed at least three times with fresh PBS, whereas before step (iii) rinsing was repeated at least six times and the solution let rest for 1 hour. In step (ii) and (iv), the two surfaces were let immersed in the solution, out of contact, for 30 minutes prior to beginning of the friction measurements. For each of the five steps, the tribological experiment (i.e. force ramp) was run twice. For better visualization, the two values of a given sample and condition were averaged in the plots ("internal averages").

### Results:

The changes in the frictional response of fresh and frozen cartilage exposed to two test solutions of synthetic lipids and hyaluronic acid, respectively, have been investigated within a comparative protocol where the same sample has been exposed alternatively to either control (buffer) or test solutions (control - test solution - control - test solution - control).

In general, the CoF in the synthetic lipids solution was lower than the one measured for HA (FIG. 8).

Taken together, the outcome suggests that the addition of synthetic lipids results in a larger reduction of frictional force than observed in the case of hyaluronic acid.

### EXAMPLE 8

### Wear measurements

### Methods:

A pin-on-disc (POD) test to evaluate the wear performance of polyethylene (PE) test pins when using the exemplary sterile composition prepared as described in Example 1 (also referred to herein as "AqueousJoint"). Three stations contained the sterile composition, diluted to a 1 : 1 ratio with bovine calf serum lubricant. Three stations were used as control and contained bovine calf serum lubricant only. Testing was conducted for 2.0 million cycles (Mc) and observable damage was reported on all test pins following testing. Testing was effected according to ASTM F732-00 and OIC's test protocol, 21091-P01 Rev A. Prior to testing, all pins and discs were marked with a unique identifier. Discs were labeled 1-6 and installed in the corresponding station number. Prior to test initiation, all discs were polished to an average roughness (Ra) ≤ 0.05 ± 0.006 µm. Three scans were taken per disc, with average values reported in 8.1 to confirm compliance to average roughness specifications. Digital images were taken of the articulating surface of the pins and discs. Following test completion, digital images were taken of the pin and disc surfaces to compare the surfaces pre- and post-test. Qualitative analyses were performed macroscopically on all the components to determine the damage features present. Microscopic images were taken of the PE pin surface to characterize the damage features that were present.

### Results:

Stations with AqueousJoint had an average wear of 5.487 ± 0.693 mg after 2.0 Mc, compared to the standard bovine calf serum lubricant which had an average wear of 25.732 ± 0.361 mg after 2.0 Mc (FIG. 9). No visible difference was noticed in the damage on the pins after testing.

## Claims

1. A sterile composition comprising an aqueous carrier and liposomes, wherein said liposomes comprise:
a) at least one bilayer-forming lipid; and
b) a polymeric compound having the general formula I: 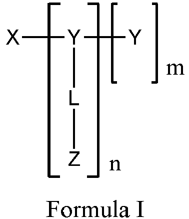
wherein:
m is zero or a positive integer;
n is an integer which is at least 1, wherein when X does not comprise a phosphate group, n is at least 2;
X is a lipid moiety;
Y is a backbone unit which forms a polymeric backbone;
L is absent or is a linking moiety; and
Z has the general formula II: 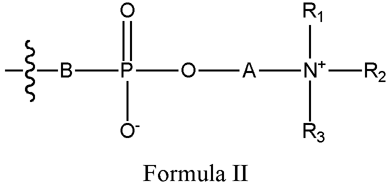
wherein:
A is a substituted or unsubstituted hydrocarbon;
B is an oxygen atom or is absent; and
R₁-R₃ are each independently selected from the group consisting of hydrogen, alkyl, cycloalkyl, heteroalicyclic, aryl and heteroaryl,
wherein a polydispersity index of said liposomes is no more than 0.2 and/or a zeta potential of said liposomes is in a range of from -40 mV to 40 mV or of from -10 mV to 10 mV,
and wherein a mean diameter and/or a zeta potential of said liposomes changes by no more than 20 % over the course of 300 days, at room temperature,
the sterile composition being **characterized by**:
an absence of observable microorganism growth upon testing in fluid thioglycollate medium at a temperature in a range of from 30 to 35 °C for up to 3 days, and/or testing the composition in soybean-casein digest medium at a temperature in a range of from 20 to 25 °C for up to 5 days, and/or when tested for sterility according to U.S. Pharmacopeia General Chapter 71; and/or
a concentration of bacterial endotoxins below a 35 endotoxin units (EU) per ml, as determined using horseshoe crab (*Limulus*) amebocyte lysate according to Chapter 85 of the U.S. Pharmacopeia.

2. The composition of claim 1, wherein Y has the formula -CR₄R₅-CR₆D-, wherein:
when Y is a backbone unit which is not attached to said L or said Z, D is R₇; and when Y is a backbone unit which is attached to said L or said Z, D is a covalent bond or a linking group attaching Y to said L or said Z, said linking group being selected from the group consisting of -O-, -S-, alkylene, arylene, sulfinyl, sulfonyl, phosphate, phosphonyl, phosphinyl, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino; and
R₄-R₇ are each independently selected from the group consisting of hydrogen, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, azo, phosphate, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino.

3. The composition of claim 1 or 2, wherein X has the general formula III: 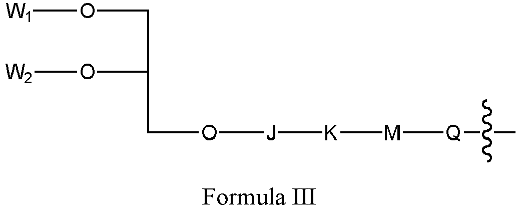 wherein:
W₁ and W₂ are each independently selected from the group consisting of hydrogen, alkyl, alkenyl, alkynyl and acyl, wherein at least one of W₁ and W₂ is not hydrogen;
J is -P(=O)(OH)-O- or absent;
K is a substituted or unsubstituted hydrocarbon from 1 to 10 carbon atoms in length, or absent;
M is a linking group selected from the group consisting of -O-, -S-, amino, sulfinyl, sulfonyl, phosphate, phosphonyl, phosphinyl, carbonyl, thiocarbonyl, urea, thiourea, carbamyl, thiocarbamyl, amido, carboxy, and sulfonamide, or absent; and
Q is a substituted or unsubstituted hydrocarbon from 1 to 10 carbon atoms in length,
wherein when M is absent, K is also absent.

4. The composition of claim 3, wherein J is -P(=O)(OH)-O- and K is selected from the group consisting of an ethanolamine moiety, a serine moiety, a glycerol moiety and an inositol moiety.

5. The composition of claims 3 or 4, wherein M is amido.

6. The composition of any one of claims 3 to 5, wherein Q is dimethylmethylene (-C(CH₃)₂-).

7. The composition of any one of claims 3 to 6, wherein at least one of W₁ and W₂ is alkyl, alkenyl, alkynyl or acyl, being from 10 to 30 carbon atoms in length.

8. The composition of any one of claims 1 to 7, for use in the treatment of a synovial joint disorder, wherein the treatment comprises intra-articular administration of the composition.

9. The composition of any one of claims 1 to 7, comprising an article of manufacture immersed therein.

10. The composition of claim 9, wherein said article of manufacture comprises a contact lens.

11. A process for preparing the sterile composition of any one of claims 1 to 10, the process comprising:
(i) providing an aqueous composition comprising said aqueous carrier and liposomes comprising said at least one bilayer-forming lipid and said polymeric compound; and
(ii) subjecting said aqueous composition to a temperature of over 100 °C, to thereby obtain the sterile composition.

12. A process for preparing a sterile article of manufacture having lipids attached to at least a portion of a surface thereof, the process comprising:
(i) contacting at least a portion of a surface of the article of manufacture with an aqueous composition comprising an aqueous carrier and liposomes, to thereby obtain an article of manufacture having lipids attached to at least a portion of a surface thereof, said liposomes comprising:
a) at least one bilayer-forming lipid; and
b) a polymeric compound having the general formula I: 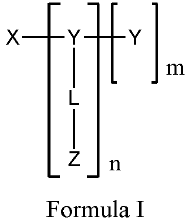 wherein:
m is zero or a positive integer;
n is an integer which is at least 1, wherein when X does not comprise a phosphate group, n is at least 2;
X is a lipid moiety;
Y is a backbone unit which forms a polymeric backbone;
L is absent or is a linking moiety; and
Z has the general formula II: 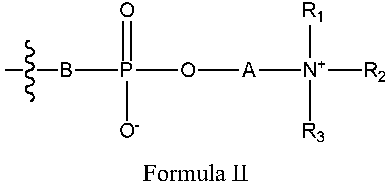
wherein:
A is a substituted or unsubstituted hydrocarbon;
B is an oxygen atom or is absent; and
R₁-R₃ are each independently selected from the group consisting of hydrogen, alkyl, cycloalkyl, heteroalicyclic, aryl and heteroaryl; and
(ii) subjecting said article of manufacture having lipids attached to at least a portion of surface thereof to a temperature of over 100 °C, to thereby obtain the sterile article of manufacture having lipids attached to at least a portion of a surface thereof,
the sterile article of manufacture being **characterized by**:
an absence of observable microorganism growth upon testing in fluid thioglycollate medium at a temperature in a range of from 30 to 35 °C for up to 3 days, and/or testing the composition in soybean-casein digest medium at a temperature in a range of from 20 to 25 °C for up to 5 days, and/or when tested for sterility according to U.S. Pharmacopeia General Chapter 71; and/or
a concentration of bacterial endotoxins below a 35 endotoxin units (EU) per ml, as determined using horseshoe crab (*Limulus*) amebocyte lysate according to Chapter 85 of the U.S. Pharmacopeia.

13. The process of claim 12, wherein Y is a substituted or unsubstituted alkylene unit.

14. The process of claim 12 or 13, wherein X has the general formula III: 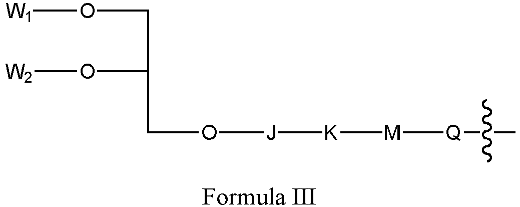 wherein:
W₁ and W₂ are each independently selected from the group consisting of hydrogen, alkyl, alkenyl, alkynyl and acyl, wherein at least one of W₁ and W₂ is not hydrogen;
J is -P(=O)(OH)-O- or absent;
K is a substituted or unsubstituted hydrocarbon from 1 to 10 carbon atoms in length, or absent;
M is a linking group selected from the group consisting of -O-, -S-, amino, sulfinyl, sulfonyl, phosphate, phosphonyl, phosphinyl, carbonyl, thiocarbonyl, urea, thiourea, carbamyl, thiocarbamyl, amido, carboxy, and sulfonamide, or absent; and
Q is a substituted or unsubstituted hydrocarbon from 1 to 10 carbon atoms in length,
wherein when M is absent, K is also absent.

15. The process of claim 14, wherein said article of manufacture comprises a contact lens.

## Patentansprüche

1. Sterile Zusammensetzung, umfassend einen wässrigen Trägerstoff und Liposome, wobei die Liposome umfassen:
a) mindestens ein doppelschichtbildendes Lipid; und
b) eine polymere Verbindung mit der allgemeinen Formel I: 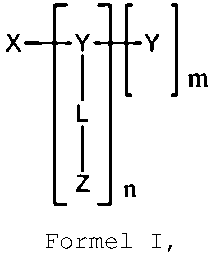
wobei:
m null oder eine positive ganze Zahl ist;
n eine ganze Zahl ist, die mindestens 1 ist, wobei, wenn X keine Phosphatgruppe umfasst, n mindestens 2 ist;
X ein Lipid-Anteil ist;
Y eine Hauptketteneinheit ist, die eine polymere Hauptkette bildet;
L fehlt oder ein Verknüpfungsanteil ist; und
Z die allgemeine Formel II aufweist: 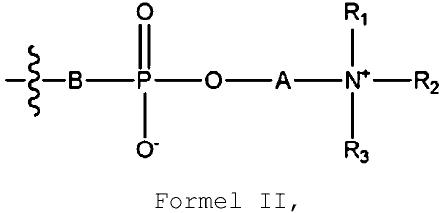
wobei:
A ein substituierter oder unsubstituierter Kohlenwasserstoff ist;
B ein Sauerstoffatom ist oder fehlt; und
R₁-R₃ jeweils unabhängig aus der Gruppe bestehend aus Wasserstoff, Alkyl, Cycloalkyl, einem Heteroalicyclus, Aryl und Heteroaryl ausgewählt sind,
wobei ein Polydispersitätsindex der Liposome nicht mehr als 0,2 beträgt und/oder ein Zetapotenzial der Liposome in einem Bereich von -40 mV bis 40 mV oder von -10 mV bis 10 mV liegt,
und wobei sich ein mittlerer Durchmesser und/oder ein Zetapotenzial der Liposome um nicht mehr als 20 % über den Verlauf von 300 Tagen bei Raumtemperatur verändern,
wobei die sterile Zusammensetzung **gekennzeichnet ist durch**:
ein Fehlen eines beobachtbaren Mikroorganismenwachstums bei Testen in flüssigem Thioglykollatmedium bei einer Temperatur in einem Bereich von 30 bis 35°C für bis zu 3 Tage
ein Ausbleiben von beobachtbarem Mikroorganismenwachstum bei der Prüfung in flüssigem Thioglycollat-Medium bei einer Temperatur im Bereich von 30 bis 35 °C für bis zu 3 Tage
und/oder Testen der Zusammensetzung in Sojakasein-Verdaumedium bei einer Temperatur in einem Bereich von 20 bis 25°C für bis zu 5 Tage und/oder wenn gemäß dem Allgemeinen Kapitel 71 der US-Pharmakopöe auf Sterilität getestet wird; und/oder
eine Konzentration von bakteriellen Endotoxinen unter 35 Endotoxin-Einheiten (EU) pro ml, wie unter Verwendung von Amöbozytenlysat des Pfeilschwanzkrebses (Limulus) gemäß Kapitel 85 der US-Pharmakopöe bestimmt.

2. Zusammensetzung nach Anspruch 1, wobei Y die Formel -CR₄R₅-CR₆D- aufweist, wobei:
wenn Y eine Hauptketteneinheit ist, die nicht an das L oder das Z angelagert ist, D R₇ ist; und wenn Y eine Hauptketteneinheit ist, die an das L oder das Z angelagert ist, D eine kovalente Bindung oder eine Verknüpfungsgruppe ist, die Y an das L oder das Z anlagert, wobei die Verknüpfungsgruppe aus der Gruppe bestehend aus -O-, -S-, Alkylen, Arylen, Sulfinyl, Sulfonyl, Phosphat, Phosphonyl, Phosphinyl, Carbonyl, Thiocarbonyl, Harnstoff, Thioharnstoff, O-Carbamyl, N-Carbamyl, O-Thiocarbamyl, N-Thiocarbamyl, C-Amido, N-Amido, C-Carboxy, O-Carboxy, Sulfonamido und Amino ausgewählt ist; und
R₄-R₇ jeweils unabhängig aus der Gruppe bestehend aus Wasserstoff, Alkyl, Alkenyl, Alkinyl, Cycloalkyl, Aryl, Heteroaryl, einem Heteroalicyclus, Halo, Hydroxy, Alkoxy, Aryloxy, Thiohydroxy, Thioalkoxy, Thioaryloxy, Sulfinyl, Sulfonyl, Cyano, Nitro, Azid, Azo, Phosphat, Phosphonyl, Phosphinyl, Oxo, Carbonyl, Thiocarbonyl, Harnstoff, Thioharnstoff, O-Carbamyl, N-Carbamyl, O-Thiocarbamyl, N-Thiocarbamyl, C-Amido, N-Amido, C-Carboxy, O-Carboxy, Sulfonamido und Amino ausgewählt sind.

3. Zusammensetzung nach Anspruch 1 oder 2, wobei X die allgemeine Formel III aufweist: 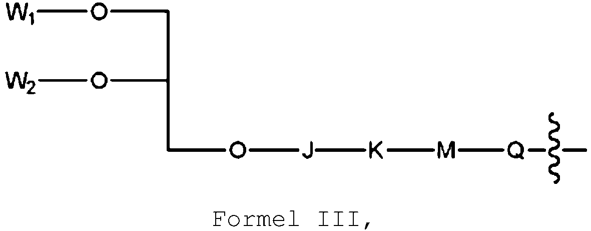 wobei:
W₁ und W₂ jeweils unabhängig aus der Gruppe bestehend aus Wasserstoff, Alkyl, Alkenyl, Alkinyl und Acyl ausgewählt sind, wobei mindestens eines von W₁ und W₂ nicht Wasserstoff ist;
J -P(=O)(OH)-O- ist oder fehlt;
K ein substituierter oder unsubstituierter Kohlenwasserstoff mit einer Länge von 1 bis 10 Kohlenstoffatomen ist oder fehlt;
M eine Verknüpfungsgruppe ist, die aus der Gruppe bestehend aus -O-, -S-, Amino, Sulfinyl, Sulfonyl, Phosphat, Phosphonyl, Phosphinyl, Carbonyl, Thiocarbonyl, Harnstoff, Thioharnstoff, Carbamyl, Thiocarbamyl, Amido, Carboxy und Sulfonamid ausgewählt ist, oder fehlt; und
Q ein substituierter oder unsubstituierter Kohlenwasserstoff mit einer Länge von 1 bis 10 Kohlenstoffatomen ist,
wobei, wenn M fehlt, K ebenfalls fehlt.

4. Zusammensetzung nach Anspruch 3, wobei J - P(=O)(OH)-O- ist und K aus der Gruppe bestehend aus einem Ethanolamin-Anteil, einem Serin-Anteil, einem Glycerin-Anteil und einem Inosit-Anteil ausgewählt ist.

5. Zusammensetzung nach den Ansprüchen 3 oder 4, wobei M Amido ist.

6. Zusammensetzung nach einem der Ansprüche 3 bis 5, wobei Q Dimethylmethylen (-C(CH₃)₂-) ist.

7. Zusammensetzung nach einem der Ansprüche 3 bis 6, wobei mindestens eines von W₁ und W₂ Alkyl, Alkenyl, Alkinyl oder Acyl ist, das eine Länge von 10 bis 30 Kohlenstoffatomen aufweist.

8. Zusammensetzung nach einem der Ansprüche 1 bis 7 zur Verwendung in der Behandlung einer Erkrankung des Synovialgelenks, wobei die Behandlung eine intraartikuläre Verabreichung der Zusammensetzung umfasst.

9. Zusammensetzung nach einem der Ansprüche 1 bis 7, umfassend einen darin eingetauchten Herstellungsgegenstand.

10. Zusammensetzung nach Anspruch 9, wobei der Herstellungsgegenstand eine Kontaktlinse umfasst.

11. Verfahren zur Herstellung der sterilen Zusammensetzung nach einem der Ansprüche 1 bis 10, wobei das Verfahren umfasst:
(i) Bereitstellen einer wässrigen Zusammensetzung, umfassend den wässrigen Trägerstoff und Liposome, umfassend das mindestens eine doppelschichtbildende Lipid und die polymere Verbindung; und
(ii) Aussetzen der wässrigen Zusammensetzung einer Temperatur von mehr als 100°C, um dadurch die sterile Zusammensetzung zu erhalten.

12. Verfahren zur Herstellung eines sterilen Herstellungsgegenstands mit an mindestens einen Abschnitt einer Oberfläche davon angelagerten Lipiden, wobei das Verfahren umfasst:
(i) Inkontaktbringen mindestens eines Abschnitts einer Oberfläche des Herstellungsgegenstands mit einer wässrigen Zusammensetzung, umfassend einen wässrigen Trägerstoff und Liposome, um dadurch einen Herstellungsgegenstand mit an mindestens einen Abschnitt einer Oberfläche davon angelagerten Lipiden zu erhalten, wobei die Liposome umfassen:
a) mindestens ein doppelschichtbildendes Lipid; und
b) eine polymere Verbindung mit der allgemeinen Formel I: 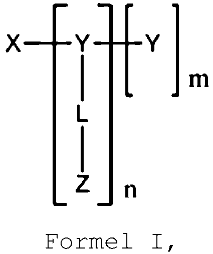 wobei:
m null oder eine positive ganze Zahl ist;
n eine ganze Zahl ist, die mindestens 1 ist, wobei, wenn X keine Phosphatgruppe umfasst, n mindestens 2 ist;
X ein Lipid-Anteil ist;
Y eine Hauptketteneinheit ist, die eine polymere Hauptkette bildet;
L fehlt oder ein Verknüpfungsanteil ist; und
Z die allgemeine Formel II aufweist: 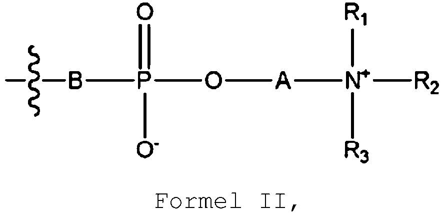
wobei:
A ein substituierter oder unsubstituierter Kohlenwasserstoff ist;
B ein Sauerstoffatom ist oder fehlt; und
R₁-R₃ jeweils unabhängig aus der Gruppe bestehend aus Wasserstoff, Alkyl, Cycloalkyl, einem Heteroalicyclus, Aryl und Heteroaryl ausgewählt sind; und
(ii) Aussetzen des Herstellungsgegenstands mit an mindestens einen Abschnitt einer Oberfläche davon angelagerten Lipiden einer Temperatur von mehr als 100°C, um dadurch den sterilen Herstellungsgegenstand mit an mindestens einen Abschnitt einer Oberfläche davon angelagerten Lipiden zu erhalten,
wobei der sterile Herstellungsgegenstand **gekennzeichnet ist durch**:
ein Fehlen eines beobachtbaren Mikroorganismenwachstums bei Testen in flüssigem Thioglykollatmedium bei einer Temperatur in einem Bereich von 30 bis 35°C für bis zu 3 Tage und/oder Testen der Zusammensetzung in Sojakasein-Verdaumedium bei einer Temperatur in einem Bereich von 20 bis 25°C für bis zu 5 Tage und/oder wenn gemäß dem Allgemeinen Kapitel 71 der US-Pharmakopöe auf Sterilität getestet wird; und/oder
eine Konzentration von bakteriellen Endotoxinen unter 35 Endotoxin-Einheiten (EU) pro ml, wie unter Verwendung von Amöbozytenlysat des Pfeilschwanzkrebses (Limulus) gemäß Kapitel 85 der US-Pharmakopöe bestimmt.

13. Verfahren nach Anspruch 12, wobei Y eine substituierte oder unsubstituierte Alkylen-Einheit ist.

14. Verfahren nach Anspruch 12 oder 13, wobei X die allgemeine Formel III aufweist: 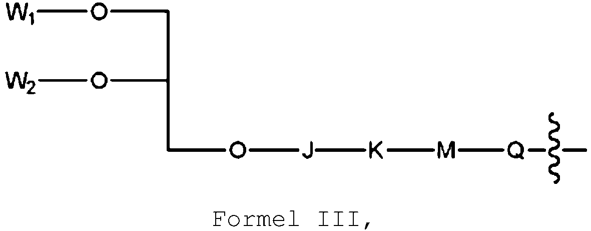 wobei:
W₁ und W₂ jeweils unabhängig aus der Gruppe bestehend aus Wasserstoff, Alkyl, Alkenyl, Alkinyl und Acyl ausgewählt sind, wobei mindestens eines von W₁ und W₂ nicht Wasserstoff ist;
J -P(=O)(OH)-O- ist oder fehlt;
K ein substituierter oder unsubstituierter Kohlenwasserstoff mit einer Länge von 1 bis 10 Kohlenstoffatomen ist oder fehlt;
M eine Verknüpfungsgruppe ist, die aus der Gruppe bestehend aus -O-, -S-, Amino, Sulfinyl, Sulfonyl, Phosphat, Phosphonyl, Phosphinyl, Carbonyl, Thiocarbonyl, Harnstoff, Thioharnstoff, Carbamyl, Thiocarbamyl, Amido, Carboxy und Sulfonamid ausgewählt ist, oder fehlt; und
Q ein substituierter oder unsubstituierter Kohlenwasserstoff mit einer Länge von 1 bis 10 Kohlenstoffatomen ist,
wobei, wenn M fehlt, K ebenfalls fehlt.

15. Verfahren nach Anspruch 14, wobei der Herstellungsgegenstand eine Kontaktlinse umfasst.

## Revendications

1. Composition stérile comprenant un véhicule aqueux et des liposomes, dans laquelle lesdits liposomes comprennent :
a) au moins un lipide formant une bicouche ; et
b) un composé polymère répondant à la formule générale I : 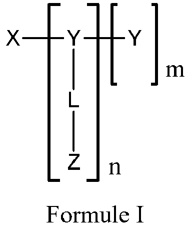
dans laquelle :
m est égal à zéro ou à un nombre entier positif ;
n est un nombre entier au moins égal à 1, n étant au moins égal à 2 lorsque X ne comporte pas de groupe phosphate ;
X est un fragment lipidique ;
Y est une unité de squelette formant un squelette polymère ;
L est absent ou est un groupement de liaison ; et
Z répond à la formule générale II : 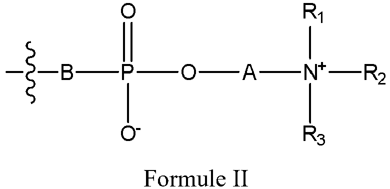
dans laquelle :
A est un hydrocarbure substitué ou non substitué ;
B est un atome d'oxygène ou est absent ; et
R₁ à R₃ sont choisis chacun indépendamment parmi le groupe constitué de l'hydrogène, d'un groupe alkyle, cycloalkyle, hétéroalicyclique, aryle et hétéroaryle,
dans laquelle l'indice de polydispersité desdits liposomes n'est pas supérieur à 0,2 et/ou le potentiel zêta desdits liposomes se situe dans une plage allant de -40 mV à 40 mV ou de -10 mV à 10 mV,
et dans lequel le diamètre moyen et/ou le potentiel zêta desdits liposomes ne varient pas de plus de 20 % au cours de 300 jours, à température ambiante,
la composition stérile étant **caractérisée par** :
l'absence de croissance observable de micro-organismes lors d'un test dans un milieu fluide au thioglycolate à une température comprise entre 30 et 35 °C pendant une durée maximale de 3 jours, et/ou lors d'un test de la composition dans un milieu de digestion à base de caséine de soja à une température comprise entre 20 et 25 °C pendant une durée maximale de 5 jours, et/ou lors d'un test de stérilité réalisé conformément au chapitre général 71 de la Pharmacopée américaine; et/ou
une concentration en endotoxines bactériennes inférieure à 35 unités d'endotoxine (UE) par ml, déterminée à l'aide de lysat d'amébocytes de crabe fer à cheval (Limulus) conformément au chapitre 85 de la Pharmacopée américaine.

2. Composition selon la revendication 1, dans laquelle Y répond à la formule -CR₄R₅ -CR₆D-, où :
lorsque Y est un motif de squelette qui n'est pas lié audit L ou audit Z, D représente R₇; et lorsque Y est un motif de squelette qui est lié audit L ou audit Z, D est une liaison covalente ou un groupe de liaison fixant Y audit L ou audit Z, ledit groupe de liaison étant choisi parmi le groupe constitué de -O-, -S-, alkylène, arylène, sulfinyle, sulfonyle, phosphate, phosphonyle, phosphinyle, carbonyle, thiocarbonyle, urée, thiourée, O-carbamyle, N-carbamyle, O-thiocarbamyle, N-thiocarbamyle, C-amido, N-amido, C-carboxy, O-carboxy, sulfonamido et amino ; et
R₄-R₇ sont chacun choisis indépendamment parmi le groupe constitué de l'hydrogène, d'un groupe alkyle, alcényle, alcynyle, cycloalkyle, aryle, hétéroaryle, hétéroalicyclique, halogéno, hydroxy, alcoxy, aryloxy, thiohydroxy, thioalcoxy, thioaryloxy, sulfinyle, sulfonyle, cyano, nitro, azide, azo, phosphate, phosphonyle, phosphinyle, oxo, carbonyle, thiocarbonyle, urée, thiourée, O-carbamyle, N-carbamyle, O-thiocarbamyle, N-thiocarbamyle, C-amido, N-amido, C-carboxy, O-carboxy, sulfonamido et amino.

3. Composition selon l'une quelconque des revendications 1 ou 2, dans laquelle X répond à la formule générale III : 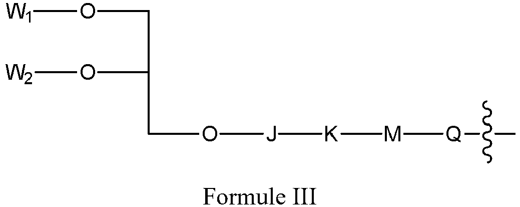 dans laquelle :
W₁ et W₂ sont chacun choisis indépendamment dans le groupe constitué par l'hydrogène, un groupe alkyle, alcényle, alcynyle et acyle, au moins l'un parmi W₁ et W₂ n'étant pas un atome d'hydrogène ;
J représente -P(=O)(OH)-O- ou est absent ;
K est un hydrocarbure substitué ou non substitué comportant de 1 à 10 atomes de carbone, ou est absent ;
M est un groupe de liaison choisi parmi le groupe constitué de -O-, -S-, amino, sulfinyle, sulfonyle, phosphate, phosphonyle, phosphinyle, carbonyle, thiocarbonyle, urée, thiourée, carbamyle, thiocarbamyle, amido, carboxy et sulfonamide, ou est absent ; et
Q est un groupe hydrocarboné substitué ou non substitué comportant de 1 à 10 atomes de carbone,
lorsque M est absent, K est également absent.

4. Composition selon la revendication 3, dans laquelle J représente -P(=O)(OH)-O- et K est choisi parmi le groupe constitué d'un groupement éthanolamine, d'un groupement sérine, d'un groupement glycérol et d'un groupement inositol.

5. Composition selon l'une quelconque des revendications 3 ou 4, dans laquelle M est un groupe amido.

6. Composition selon l'une quelconque des revendications 3 à 5, dans laquelle Q représente un groupe diméthylméthylène (-C(CH₃ )₂-).

7. Composition selon l'une quelconque des revendications 3 à 6, dans laquelle au moins l'un des groupes W₁ et W₂ est un groupe alkyle, alcényle, alcynyle ou acyle, comportant de 10 à 30 atomes de carbone.

8. Composition selon l'une quelconque des revendications 1 à 7, destinée à être utilisée dans le traitement d'un trouble de l'articulation synoviale, dans laquelle le traitement comprend l'administration intra-articulaire de la composition.

9. Composition selon l'une quelconque des revendications 1 à 7, comprenant un article manufacturé immergé dans celle-ci.

10. Composition selon la revendication 9, dans laquelle ledit article manufacturé comprend une lentille de contact.

11. Procédé de préparation de la composition stérile selon l'une quelconque des revendications 1 à 10, le procédé comprenant :
(i) la mise à disposition d'une composition aqueuse comprenant ledit véhicule aqueux et des liposomes comprenant ledit au moins un lipide formant une bicouche et ledit composé polymère; et
(ii) le fait de soumettre ladite composition aqueuse à une température supérieure à 100 °C, afin d'obtenir ainsi la composition stérile.

12. Procédé de préparation d'un article manufacturé stérile comportant des lipides fixés sur au moins une partie de sa surface, le procédé comprenant :
(i) la mise en contact d'au moins une partie de la surface de l'article manufacturé avec une composition aqueuse comprenant un véhicule aqueux et des liposomes, afin d'obtenir ainsi un article manufacturé comportant des lipides fixés sur au moins une partie de sa surface, lesdits liposomes comprenant :
a) au moins un lipide formant une bicouche ; et
b) un composé polymère répondant à la formule générale I : 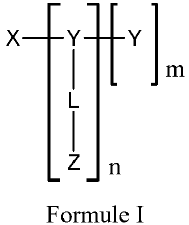 dans laquelle :
m est égal à zéro ou à un nombre entier positif ;
n est un nombre entier au moins égal à 1, n étant au moins égal à 2 lorsque X ne comporte pas de groupe phosphate ;
X est un fragment lipidique ;
Y est une unité de squelette formant un squelette polymère ;
L est absent ou est un groupement de liaison ; et
Z répond à la formule générale II : 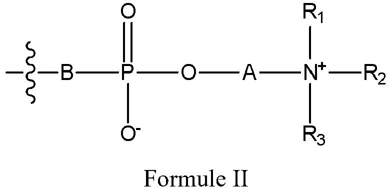
dans laquelle :
A est un hydrocarbure substitué ou non substitué ;
B est un atome d'oxygène ou est absent ; et
R₁ à R₃ sont chacun choisis indépendamment parmi le groupe constitué de l'hydrogène, d'un groupe alkyle, cycloalkyle, hétéroalicyclique, aryle et hétéroaryle ; et
(ii) soumettre ledit article manufacturé, sur au moins une partie de la surface duquel sont fixés des lipides, à une température supérieure à 100 °C, afin d'obtenir ainsi l'article manufacturé stérile, sur au moins une partie de la surface duquel sont fixés des lipides,
ledit article manufacturé stérile étant **caractérisé par** :
l'absence de croissance observable de micro-organismes lors d'un test dans un milieu fluide au thioglycollate à une température comprise entre 30 et 35 °C pendant une durée pouvant aller jusqu'à 3 jours, et/ou lors d'un test de la composition dans un milieu de digestion à base de soja et de caséine à une température comprise entre 20 et 25 °C pendant une durée pouvant aller jusqu'à 5 jours, et/ou lors d'un test de stérilité réalisé conformément au chapitre général 71 de la Pharmacopée américaine ; et/ou
une concentration en endotoxines bactériennes inférieure à 35 unités d'endotoxine (UE) par ml, déterminée à l'aide de lysat d'amébocytes de crabe fer à cheval (Limulus) conformément au chapitre 85 de la Pharmacopée américaine.

13. Procédé selon la revendication 12, dans lequel Y est un motif alkylène substitué ou non substitué.

14. Procédé selon l'une quelconque des revendications 12 ou 13, dans lequel X répond à la formule générale III : 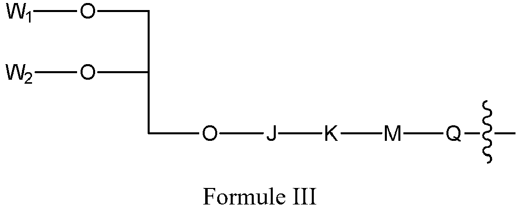 dans laquelle :
W₁ et W₂ sont chacun choisis indépendamment dans le groupe constitué par l'hydrogène, un groupe alkyle, alcényle, alcynyle et acyle, au moins l'un parmi W₁ et W₂ n'étant pas un atome d'hydrogène ;
J représente -P(=O)(OH)-O- ou est absent ;
K est un hydrocarbure substitué ou non substitué comportant de 1 à 10 atomes de carbone, ou est absent ;
M est un groupe de liaison choisi parmi le groupe constitué de -O-, -S-, amino, sulfinyle, sulfonyle, phosphate, phosphonyle, phosphinyle, carbonyle, thiocarbonyle, urée, thiourée, carbamyle, thiocarbamyle, amido, carboxy et sulfonamide, ou est absent ; et
Q est un groupe hydrocarboné substitué ou non substitué comportant de 1 à 10 atomes de carbone,
lequel, lorsque M est absent, K est également absent.

15. Procédé selon la revendication 14, dans lequel ledit article manufacturé comprend une lentille de contact.
